# EUROPEAN PATENT APPLICATION

(11) **EP 4 299 596 A1**
(43) Date of publication of application: **03.01.2024**
(21) Application number: 22182414.7
(22) Date of filing: 30.06.2022
(51) Int. Cl.: C07K 17/06, G01N 33/543

(54) **MOLECULAR PROBE FUNCTIONALIZED ELEMENT**

(71) Applicant: betaSENSE GmbH, 48147 Münster (DE)
(72) Inventor: Güldenhaupt, Jörn, 59192 Bergkamen (DE); Mann, Marvin, 58540 Meinerzhagen (DE); Woitzik, Nathalie, 44137 Dortmund (DE); Langenhoff, Lennart Carl, 44789 Bochum (DE); Budde, Brian, 44791 Bochum (DE); Gerwert, Grischa, 44225 Dortmund (DE); Kötting, Carsten, 44892 Bochum (DE); Gerwert, Klaus, 48147 Münster (DE)
(74) Representative: Maiwald GmbH

(57) **Abstract**

The present invention provides a molecular probe functionalized element comprising a solid base element, a first layer comprising a linker A, a second layer comprising a peptide compound, a third layer comprising a linker B, and a fourth layer comprising a molecular probe, wherein the peptide compound is covalently bound to the solid base element via linker A and the molecular probe is covalently bound to the peptide compound via linker B. The present invention also provides a process for preparing the molecular probe functionalized element and a device comprising the molecular probe functionalized element such as an optical biosensor. Further, the present invention provides a method for detecting a biomarker comprising a) bringing into contact the molecular probe functionalized element with a sample suspected to comprise a target analyte; b) detecting the biomarker based on an interaction between the molecular probe and the target analyte. The present invention also provides a use of the molecular probe functionalized element or of the device for one or more of: a companion diagnostic test; diagnosing Alzheimer's disease, Parkinson's disease, amyotrophic lateral sclerosis, Diabetes, Huntington, Prion disease, or a tumor in a patient; monitoring of therapy of patients with Alzheimer's disease, Parkinson's disease, amyotrophic lateral sclerosis, Diabetes, Huntington, Prion disease, or a tumor; and screening of drugs for the treatment of Alzheimer's disease, Parkinson's disease, amyotrophic lateral sclerosis, Diabetes, Huntington, Prion disease, or a tumor.

## Description

### TECHNICAL FIELD OF THE INVENTION

The present invention relates to a molecular probe functionalized element and to a process for preparing a molecular probe functionalized element. The present invention further relates to a device comprising the molecular probe functionalized element. For example, the device may be, but is not limited to, a biosensor for detecting a biomarker. The present invention also relates to a method for detecting a biomarker.

### BACKGROUND OF THE INVENTION

Molecular probes are extensively used for biochemical applications, molecular biological application, medical applications, and even non-medical applications. Molecular probes such as antibodies, nucleic acids, biomolecular pairs and complexes, enzymes, drugs, ligands, small molecules, microorganisms, and the like, can interact with a desired target analyte. Target analytes can vary widely and include antibodies, proteins, protein complexes, drugs, toxins, diseases biomarkers, cells such as tumor cells, and the like. The biochemical interaction of the molecular probe with the target analyte is converted into a signal which can be detected and/or quantified according to the selected analytical technique. For example, in biosensors, the biochemical interaction of the molecular probe with the target analyte produces a physical-chemical change, which is then converted into a measurable signal through a transduction element. The transduction element can be, for example, an optical transduction element as used for surface plasmon resonance (SPR) spectroscopy, attenuated total reflection infrared (ATR-IR) spectroscopy, Raman spectroscopy, UV/Vis spectroscopy, fluorescence spectroscopy, and the like.

In many applications, the molecular probe is immobilized on the surface of a solid support of a device such as a biosensor to create a functional surface. A fluid mixture containing the target analyte is passed over the functional surface to allow for an interaction between the molecular probe and the target analyte, which is then converted into the measurable signal. For example, in an optical biosensor based on ATR-IR immunodetection, the molecular probe is immobilized on a reflection element such as a silicon, germanium, zinc selenide crystal, gold coated elements or fibers to create a functional surface on the reflection element. The interaction with the target analyte can then be measured based on the differences in the measured IR spectra.

A problem associated with functional surfaces containing immobilized molecular probes is often non-specific binding of non-target components of an analyte mixture to the surface. Non-specific binding can negatively affect signal-to-noise ratio and, in the worst case, can make a reliable detection of a target analyte impossible. Non-specific binding to a functional surface can be particularly pronounced if a molecular probe is intended to be used to detect larger proteins, lipids, or nucleic acids or other compounds present in complex fluids such as from a body fluid, e.g. CSF, intestinal fluid, ocular fluid, pulmonary fluid, blood, or the like.

Furthermore, it is often difficult to immobilize the molecular probe, e.g. an antibody, a protein, or a protein complex, on a solid support surface without loosing its native functionality. A decrease in native functionality of the molecular probe can result in a decreased analyte binding capacity which is in turn detrimental to the sensitivity and efficiency of the selected analytical technique. Another challenge is to immobilize the molecular probe on a surface in a way that detachment of the probe cannot occur, e.g. by washing of the probe during measurement in a flow chamber.

Therefore, there is a continuing need in the art for a molecular probe as part of functional surfaces, for example, for use in detecting biomarkers and/or as part of detection devices such as biosensors. In particular, it is desirable that the molecular probe functionalized element as a functional surface shows no or decreased non-specific binding, especially towards complex analyte mixtures. It is further desirable that the immobilized molecular probe is functional and stable, and is not prone to detachment from the surface.

One object of the present invention is to provide an improved molecular probe-functionalized element.

### SUMMARY OF THE INVENTION

In one aspect, the present invention provides a molecular probe functionalized element according to independent claim 1. The molecular probe functionalized element comprises
a solid base element,
a first layer comprising a linker A,
a second layer comprising a peptide compound,
a third layer comprising a linker B, and
a fourth layer comprising a molecular probe,
wherein the peptide compound is covalently bound to the base element via linker A and the molecular probe is covalently bound to the peptide compound via linker B.

The inventors have surprisingly found that the molecular probe functionalized element according to the invention provides a functional surface which is highly stable while maintaining the activity of the molecular probe. The functional surface shows decreased non-specific binding and has an increased target analyte binding capacity. While there is often a trade-off in the prior art between good blocking of a surface and activity of a molecular probe, the molecular probe functionalized element provided by the invention achieves a good blocking and a good activity of the molecular probe. The signal-to-noise ratio can be improved when using the inventive molecular probe functionalized element.

In particular, the inventors found that a more native and functional molecular probe can be immobilized on the surface by (i) covalently blocking the surface of the base element with a peptide compound before (ii) covalently attaching the molecular probe (e.g. an antibody) via a linker to the peptide compound. Furthermore, it has been found that less non-specific binding from an analyte mixture (e.g. a body fluid) occurs on the peptide-blocked surface.

Thus, the peptide compounds of the second layer on the one hand can form a peptide blocking layer on the surface of the base element to reduce non-specific binding, and on the other hand can serve as a platform for stably linking the molecular probe to the base element by a linker compound. Therefore, the molecular probe functionalized element according to the invention is particularly useful for detecting a biomarker and/or is particularly useful for a device for detecting a biomarker such as optical biosensor, e.g. a biosensor based on ATR-IR immunodetection.

Additionally, the molecular probe functionalized element can be prepared in a comparatively simple process under ambient conditions.

In one aspect, the present invention provides a device, preferably a biosensor, wherein the device comprises a molecular probe functionalized element according to the invention.

In one aspect, the present invention provides a process for preparing a molecular probe functionalized element according to the invention. The process comprises the steps of:
a) providing a solid base element;
b) preparing a linker A precursor which is covalently bound to the surface of the solid base element;
c) reacting the linker A precursor with a peptide compound to covalently bind the peptide compound to the solid base element via linker A;
d) preparing a linker B precursor which is covalently bound the peptide compound; and
e) reacting the linker B precursor with a molecular probe to covalently bind the molecular probe to the peptide compound via linker B.

In one aspect, the present invention provides a method for detecting a biomarker. The method comprises
a) bringing into contact the molecular probe functionalized element according to the invention with a sample suspected to comprise a target analyte, and optionally wherein the sample is a blood or CSF sample;
b) detecting the biomarker based on an interaction between the molecular probe and the target analyte, and optionally wherein the interaction between the molecular probe and the target analyte is detected by infrared spectroscopy, and optionally wherein the biomarker is an amide band maximum of the target analyte.

In one aspect, the present invention provides a use of the molecular probe functionalized element according to the invention or of the device according to the invention for one or more of:
a companion diagnostic test,
diagnosing Alzheimer's disease, Parkinson's disease, amyotrophic lateral sclerosis, Diabetes, Huntington, Prion disease, or a tumor in a patient,
monitoring of therapy of patients with Alzheimer's disease, Parkinson's disease, amyotrophic lateral sclerosis , Diabetes, Huntington, Prion disease, or a tumor, and
screening of drugs for the treatment of Alzheimer's disease, Parkinson's disease, amyotrophic lateral sclerosis , Diabetes, Huntington, Prion disease, or a tumor.

A "peptide compound" in the meaning of the present invention is a compound comprising at least two amino acids, and optionally at least four amino acids, which are linked by a peptide bond, and optionally by peptide bonds. In this context, "amino acid" is to be understood in a broad way in that it is a compound comprising a carboxylic acid group and an amino group.

Where the present description and claims define subject-matter "comprising" certain features, this is to be interpreted as meaning the subject-matter includes those features, but that it does not exclude other non-specified features. For the purposes of the present invention, the term "essentially consisting of" and "consisting of" are considered to be optional embodiments of the term "comprising of". If hereinafter a subject-matter is defined to comprise at least a certain number of features, this is also to be understood to disclose a subject-matter, which optionally (essentially) consists only of these features.

Whenever the terms "including" or "having" are used, these terms are meant to be equivalent to "comprising" as defined above.

Where an indefinite or definite article is used when referring to a singular noun, e.g. "a", "an" or "the", this includes a plural of that noun unless something else is specifically stated. Terms like "obtainable" or "obtained" are used interchangeably. This e.g. means that, unless the context clearly dictates otherwise, the term "obtained" does not mean to indicate that, e.g., an embodiment must be obtained by, e.g., the sequence of steps following the term "obtained" even though such a limited understanding is always included by the terms "obtained" as a preferred embodiment.

Preferred embodiments of the invention are defined in the dependent claims.

### DETAILED DESCRIPTION OF THE INVENTION

In the following, the present invention is described in more detail.

### The molecular probe functionalized element according to the invention

In one aspect, the present invention provides a molecular probe functionalized element comprising
a solid base element,
a first layer comprising a linker A,
a second layer comprising a peptide compound,
a third layer comprising a linker B, and
a fourth layer comprising a molecular probe,
wherein the peptide compound is covalently bound to the base element via linker A and the molecular probe is covalently bound to the peptide compound via linker B.

According to one embodiment, the molecular probe functionalized element is obtained or obtainable by a process according to the invention.

According to one embodiment, a molecular probe functionalized element is provided which consists of the solid base element, the first layer, the second layer, the third layer, and the fourth layer as defined herein.

The molecular probe functionalized element according to the invention can be understood in that it has a structure, wherein a plurality of linkers A, and optionally a matrix compound, forms a first layer on the surface of the base element, a plurality of peptide compounds forms a second layer on the first layer, a plurality of linker B forms a third layer on the second layer, and a plurality of molecular probes forms a fourth layer on the third layer. The first and the third layer comprising the linkers A and B, respectively, can be understood as linkage layers which link the solid base element to the peptide compound of the second layer and the peptide compound to the molecular probe. The second layer comprising the peptide compound can have the function of a blocking layer.

The term "layer" in the context of the present invention is not to be construed as restricting the first layer, second layer, third layer, and fourth layer to specific dimensions or to a specific aggregation state.

### Solid base element

The molecular probe functionalized element according to the present invention comprises a solid base element. The base element is a solid support which is covalently bound to linker A.

The solid base element may be a solid support which is suitable for use in a device for detecting a biomarker. The solid base element may be a solid support which is suitable for use in biosensors such as optical biosensors, acoustic biosensors or field-effect biosensors. The solid base element may be a quartz crystal microbalance (QCM), a semi-conductor or an optical element suitable for use in an optical biosensor.

According to one embodiment, the solid base element is a waveguide (e.g. a SPR waveguide or an ATR waveguide) which is suitable for use in an optical biosensor. According to one exemplary embodiment, the solid base element is an internal reflection element. According to one preferred embodiment of the invention, the solid base element is an internal reflection element which is suitable for use in ATR-IR-spectroscopy.

According to one embodiment, the solid base element is an optical element. According to one embodiment, the solid base element is an optical element which is suitable for use in ATR-IR spectroscopy, transmission IR spectroscopy, SPR spectroscopy or surface-enhanced infrared absorption (SEIRA) spectroscopy, and preferably ATR-IR spectroscopy and transmission IR spectroscopy, and more preferably ATR-IR spectroscopy.

According to one preferred embodiment, the solid base element is an IR-transparent optical element, which is preferably suitable for use in ATR-IR spectroscopy, transmission IR spectroscopy, SPR spectroscopy or surface-enhanced infrared absorption (SEIRA) spectroscopy, and more preferably ATR-IR spectroscopy and transmission IR spectroscopy, and most preferably ATR-IR spectroscopy.

Preferably, the IR-transparent optical element is at least transparent to infrared radiation with wavenumbers in a range of 40 to 13000 cm⁻¹, and more preferably in the range of 500 to 3000 cm⁻¹, like in the range of 1400 to 1800 cm⁻¹. In one specifically preferred embodiment, the optical element, preferably the IR-transparent optical element, is an ATR-IR waveguide.

According to one embodiment, the solid base element is a material and/or crystal selected from the group consisting of plastic, glass, diamond, gold, silver, palladium, germanium, silicon, silicon dioxide, silver halides, GaAs, ZnSe, ZnS, thallium(I) mixed halides (e.g. KRS-5), and AMTIRs. "AMTIR" is an amorphous material transmitting infrared radiation which is known in the art. A suitable AMTIR may be Ge₃₃As₁₂Se₅₅ (AMTIR-1), and preferably is a silicon crystal or a germanium crystal, and more preferably is a silicon crystal.

According to one preferred embodiment, the solid base element is a silicon crystal or a germanium crystal, and preferably a silicon crystal.

The solid base element may be at least partially surface-modified, e.g. by an oxide layer (e.g. silicon dioxide layer). The surface modification may aid the attachment of linker A.

According to one embodiment of the present invention, at least a part of the solid base element has an oxide surface layer, and the peptide is covalently bound to the oxide surface layer via linker A. The oxide surface layer is preferably a metal oxide surface layer or a metalloid oxide surface layer. A "metalloid oxide" is an oxide of an element selected from the group consisting of boron, silicon, germanium, arsenic, antimony and tellurium.

According to one embodiment of the present invention, at least a part of the solid base element has a silicon dioxide surface layer, and wherein the peptide is covalently bound to the silicon dioxide surface layer via linker A.

According to one preferred embodiment of the present invention, the solid base element is a silicon crystal, wherein at least a part of the silicon crystal has a silicon dioxide surface layer, and wherein the peptide compound is covalently bound to the silicon dioxide surface layer via linker A. In this case, it is further preferred that linker A comprises a silyl group which is covalently bound to the silicon dioxide surface.

The solid base element may be covalently bound to a surface attachment group of linker A. For example, the surface attachment group of linker A may be a silicon-containing group or a sulfur-containing group. The surface attachment group of linker A can be selected in view of the material of the solid base element. For example, if the solid base element is a silicon crystal which is at least partially surface-modified with silicon dioxide or a germanium crystal which is at least partially surfaceoxidized, the surface attachment group of linker A may be a silicon-containing group. If the solid base element is a germanium crystal, the surface attachment group of linker A may be a sulfur-containing group.

### First layer comprising linker A

The molecular probe functionalized element according to the present invention further comprises a first layer comprising a linker A.

Linker A covalently binds the peptide compound to the solid base element. This can be understood in that the linker A has a surface attachment group which is covalently bound to the solid base element and a peptide attachment group which is covalently bound to the peptide compound, and wherein the surface attachment group is covalently bound to the peptide attachment group via a linking moiety.

Linker A is not particularly limited in terms of its chemical structure as long as linker A is suitable for covalently binding a peptide compound as defined herein to the solid base element.

The surface attachment group of linker A can be selected in view of the material of the solid base element as defined herein above.

Linker A may comprise a surface attachment group which is a silicon- or sulfur-containing group. According to one embodiment of the invention, linker A comprises a surface attachment group which is covalently bound to the solid base element, and wherein the surface attachment group is a silicon-containing group or a sulfur-containing group.

The surface attachment group may have the formula:
BE-Y¹-/,
wherein BE indicates attachment of linker A to the solid base element;
Y¹ is S or -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element,
and "/" indicates the attachment point to the remaining part of linker A.

According to one embodiment, the solid base element is a silicon crystal, which is optionally at least partially surface-modified by silicon dioxide, wherein the surface attachment group has the formula:
BE-Y¹-/,
wherein BE indicates attachment of linker A to the solid base element;
Y¹ is -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element,
and "/" indicates the attachment point to the remaining part of linker A.

According to one embodiment, linker A comprises an alkylene oxide unit or an poly(alkylene oxide) unit. Preferably, linker A comprises an ethylene glycol unit or an polyethylene glycol unit. For example, linker A can comprise a -(OCH₂CH₂)ₖ- group, wherein k is an integer from 1 to 100, preferably from 1 to 50, and more preferably from 1 to 16.

According to one embodiment, linker A comprises a surface attachment group which is covalently bound to the solid base element and a peptide attachment group which is covalently bound to the peptide compound, and wherein the surface attachment group is covalently bound to the peptide attachment group via a linking moiety, and wherein the linking moiety comprises a -(OCH₂CH₂)ₖ- group, wherein k is an integer from 1 to 100, preferably from 1 to 50, and more preferably from 1 to 16.

Additionally or alternatively, linker A may comprise a group which is obtainable by a bioorthogonal ligation reaction. Bioorthogonal ligation reactions are known to the skilled person. The skilled person is also familiar with the chemical structures of the functional groups which are obtainable by said reactions. Bioorthogonal ligation reactions include, but are not limited to, reactions between an azide and phosphine or phosphite ("Staudinger ligation"), [2+3]-cycloadditions between azide and alkyne ("click reaction), between azide and cycloalkyne ("copper-free click reaction"), between nitrone and cycloalkyne, [2+4]-cycloadditions between tetrazine and a trans-alkene ("tetrazine ligation"), oxime/hydrazone formation from aldehyde and ketone, and the like. Hence, the bioorthogonal ligation reaction can be one of the following reactions: (i) a reaction between an azide and a phosphine or phosphite; (ii) a reaction between an azide and an alkyne (including cycloalkynes); (iii) a reaction between an azide and a cycloalkyne; (iv) a reaction between a tretrazine and a transalkenes; (v) an oxime- and/or hydrazone-forming reaction.

According to one embodiment, linker A comprises an optionally substituted N-heterocyclic group, wherein the N-heterocyclic group is selected from triazoles (e.g. 1,2,3-triazoles), dihydropyridazines, and pyridazines.

Preferably, linker A comprises an optionally substituted triazole group which is obtainable by a [2+3]-cycloaddition between an azide compound and an alkyne compound ("Click ligation") or linker A comprises an optionally substituted dihydropyridazine which is obtainable by a [2+4]-cycloaddition between an tetrazine compound and a trans-alkene compound ("Click ligation").

According to one embodiment, linker A comprises a surface attachment group which is covalently bound to the solid base element and a peptide attachment group which is covalently bound to the peptide compound, and wherein the surface attachment group is covalently bound to the peptide attachment group via a linking moiety, and wherein the linking moiety comprises a group which is obtainable by a bioorthogonal ligation reaction, and preferably wherein the linking moiety comprises an optionally substituted N-heterocyclic group, wherein the N-heterocyclic group is selected from triazoles (e.g. 1,2,3-triazoles), dihydropyridazines, and pyridazines.

Linker A may comprise a peptide attachment group, which may be any group which is suitable for attaching a linker to a peptide compound, and optionally to an amino, thiol or alcohol group of a peptide compound, and optionally to an amino group of a peptide compound (e.g. an amino group of a lysine residue).

The peptide attachment group may be an ester group, an amide group or a succinimide group or a ring-opened product of a succinimide group, and optionally is an amide group.

The peptide attachment group may be one of: 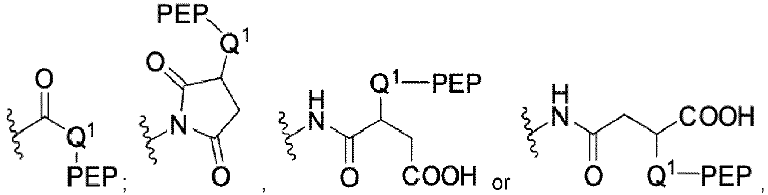 wherein Q¹ is NH, O or S of an amino acid of the peptide compound,
PEP is the peptide compound, and
the wavy line indicates the attachment point to the remaining part of linker A.

According to one embodiment of the present invention, linker A comprises
a surface attachment group which is covalently bound to the solid base element, and
a peptide attachment group which is covalently bound to the peptide compound,
wherein the surface attachment group is covalently bound to the peptide attachment group via a linking moiety;
wherein the surface attachment group is a silicon- or sulfur-containing group, and
wherein the peptide attachment group is an ester group, an amide group, or a succinimide group or a ring-opened product thereof, preferably the peptide attachment group is one of: 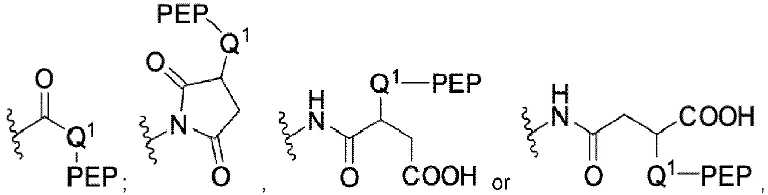 wherein Q¹ is NH, O or S of an amino acid of the peptide compound,
PEP is the peptide compound, and
the wavy line indicates the attachment point to the remaining part of linker A.

According to one embodiment of the present invention, linker A comprises
a surface attachment group which is covalently bound to the solid base element, and
a peptide attachment group which is covalently bound to the peptide compound,
wherein the surface attachment group is covalently bound to the peptide attachment group via a linking moiety;
wherein the surface attachment group is a silicon- or sulfur-containing group,
wherein the linking moiety comprises an optionally substituted N-heterocyclic group selected from triazoles (e.g. 1,2,3-triazoles), dihydropyridazines and pyridazines, which is optionally obtainable by a cycloaddition reaction (e.g. a [2+3]- or [2+4]-cycloaddition); and
wherein the peptide attachment group is an ester group, an amide group, or a succinimide group or a ring-opened product thereof, preferably the peptide attachment group is one of: 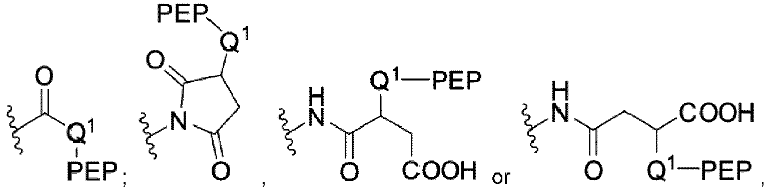
wherein Q¹ is NH, O or S of an amino acid of the peptide,
PEP is the peptide compound, and
the wavy line indicates the attachment point to the remaining part of linker A.

According to one embodiment of the present invention, linker A comprises
a surface attachment group which is covalently bound to the solid base element, and
a peptide attachment group which is covalently bound to the peptide compound,
wherein the surface attachment group is covalently bound to the peptide attachment group via a linking moiety;
wherein the surface attachment group is a silicon- or sulfur-containing group,
wherein the linking moiety comprises a -(OCH₂CH₂)ₖ- group, wherein k is an integer from 1 to 100, preferably from 1 to 50, and more preferably 1 to 16; and
wherein the peptide attachment group is an ester group, an amide group, or a succinimide group or a ring-opened product thereof, preferably the peptide attachment group is one of: 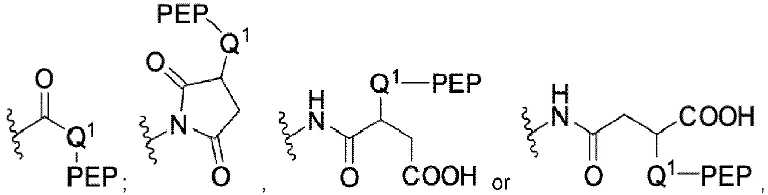
wherein Q¹ is NH, O or S of an amino acid of the peptide,
PEP is the peptide compound, and
the wavy line indicates the attachment point to the remaining part of linker A.

According to one preferred embodiment of the present invention, linker A comprises
a surface attachment group which is covalently bound to the solid base element, and
a peptide attachment group which is covalently bound to the peptide compound,
wherein the surface attachment group is covalently bound to the peptide attachment group via a linking moiety;
wherein the surface attachment group is a silicon- or sulfur-containing group,
wherein the linking moiety comprises (i) an optionally substituted N-heterocyclic group selected from triazoles (e.g. 1,2,3-triazoles), dihydropyridazines and pyridazines, which is optionally obtainable by a cycloaddition reaction (e.g. a [2+3]- or [2+4]-cycloaddition), and/or (ii) a -(OCH₂CH₂)ₖgroup, wherein k is an integer from 1 to 100, preferably 1 to 50, and more preferably 1 to 16, and preferably the linking moiety comprises feature (i) and (ii); and
wherein the peptide attachment group is an ester group, an amide group, or a succinimide group or a ring-opened product thereof, preferably the peptide attachment group is one of: 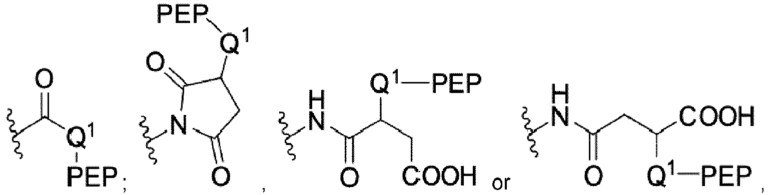
wherein Q¹ is NH, O or S of an amino acid of the peptide,
PEP is the peptide compound, and
the wavy line indicates the attachment point to the remaining part of linker A.

The inventors found that by connecting the surface of the solid base element to the peptide compound via a linker A which is sequentially prepared by click ligation, and therefore comprises an optionally substituted N-heterocyclic group selected from triazoles (e.g. 1,2,3-triazoles), dihydropyridazines and pyridazines, in its linking moiety, it is possible to provide a better and more dense functionalization of the surface of the base element.

According to one preferred embodiment of the invention, linker A has a structure according to any one of formula (LA-I) to (LA-III-b) or a salt thereof, and preferably has a structure according to formula (LA-II) or (LA-II-b) or a salt thereof: 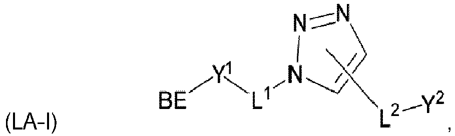 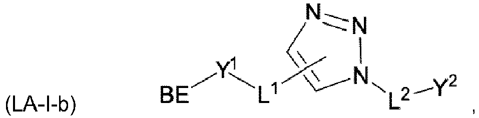 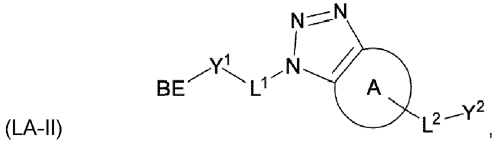 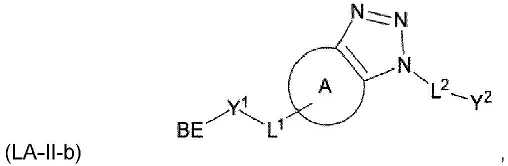 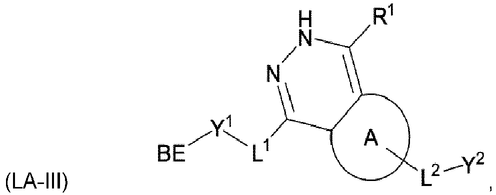 and 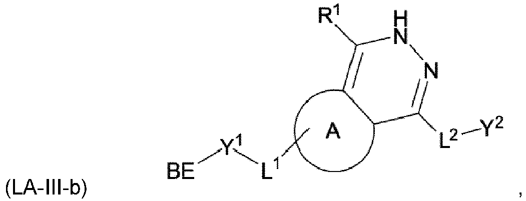 wherein
BE indicates attachment of linker A to the solid base element;
Y¹ is S or -O-Si(R²)₂-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element;
L¹ and L² are each a linking moiety;
Y² is an ester group, an amide group, or a succinimide group or a ring-opened product thereof, each of which is covalently bound to the peptide compound;
ring A, if present, is an optionally substituted 8-membered carbocycle or an optionally substituted 8-membered heterocycle (e.g. an optionally substituted 8-membered N-heterocycle); and
R¹, if present, is selected from H, an optionally substituted alkyl and an optionally substituted aryl.

According to one preferred embodiment, the linking moiety L¹ in any one of above formula (LA-I) to (LA-III-b) comprises an ethylene glycol group or a PEG group, and more preferably a (OCH₂CH₂)ₖ- group, wherein k is an integer from 1 to 100, preferably 1 to 50, and more preferably from 1 to 16.

The optional additional silane compound being attached to the surface of the solid base element may be the matrix compound as defined herein below.

According to one embodiment, linker A has a structure according to formula (LA-IV) or a salt thereof: 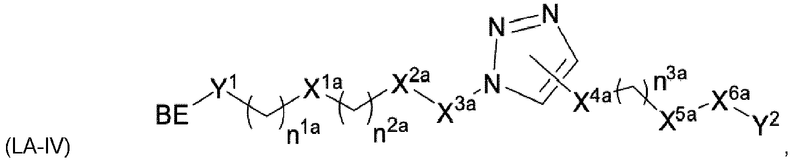 wherein
BE indicates attachment of linker A to the solid base element;
Y¹ is S or -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element;
n^{1a} is an integer from 1 to 6;
X^{1a} is a bond, -C(O)NH- or-NHC(O)-;
n^{2a} is an integer from 1 to 16;
X^{2a} is a bond or -(OCH₂CH₂)n^{4a}-, wherein n^{4a} is an integer from 1 to 16;
X^{3a} is a bond or -CH₂-;
X^{4a} is a bond;
n^{3a} is an integer from 1 to 5;
X^{5a} is a bond or -(OCH₂CH₂)n^{5a}-, wherein n^{5a} is an integer from 1 to 16;
X^{6a} is a bond or -(CH₂)n^{6a}-, wherein n^{6a} is an integer from 1 to 5; and
Y² is an ester group, an amide group, or a succinimide group or a ring-opened product thereof, each of which is covalently bound to the peptide compound.

Where a substituent may be a "bond", this means "covalent bond". As will be understood by those skilled in the art, where two or more adjacent substituents are selected to be a bond, this means that the two adjacent substituents form one bond. For example, if X^{5a} and X^{6a} are both selected to be a bond, this means X^{5a} and X^{6a} form one bond which binds the methylene group ("CH₂-") to Y². This understanding applies to each embodiment disclosed herein where two or more adjacent substituents can be selected to be a bond.

According to one preferred embodiment, linker A has a structure according to formula (LA-V) or a salt thereof: 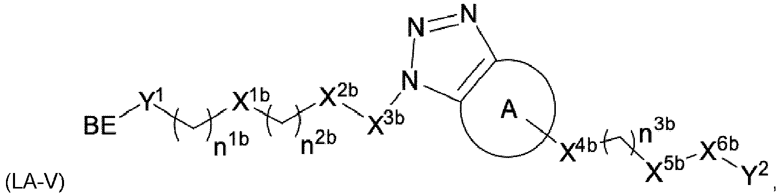 wherein
BE indicates attachment of linker A to the solid base element;
Y¹ is S or -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element;
n^{1b} is an integer from 1 to 6;
X^{1b} is a bond, -C(O)NH- or-NHC(O)-;
n^{2b} is an integer from 1 to 16;
X^{2b} is a bond or -(OCH₂CH₂)n^{4b}-, wherein n^{4b} is an integer from 1 to 16;
X^{3b} is a bond or -CH₂-;
ring A is an optionally substituted 8-membered carbocycle or an optionally substituted 8-membered heterocycle;
X^{4b} is a bond, O, -C(O)- or a carbon atom which forms a cyclopropyl group with two adjacent carbons of ring A;
n^{3b} is an integer from 1 to 5;
X^{5b} is a bond or -(OCH₂CH₂)n^{5b}-, wherein n^{5b} is an integer from 1 to 16;
X^{6b} is a bond or -(CH₂)n^{6b}-, wherein n^{6b} is an integer from 1 to 5; and
Y² is an ester group, an amide group, or a succinimide group or a ring-opened product thereof, each of which is covalently bound to the peptide compound.

According to one embodiment, linker A has a structure according to formula (LA-VI) or a salt thereof: 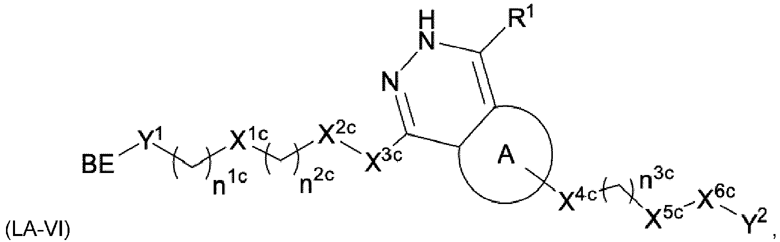 wherein
BE indicates attachment of linker A to the solid base element;
Y¹ is S or -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element;
n^{1c} is an integer from 1 to 6;
X^{1c} is a bond, -C(O)NH- or-NHC(O)-;
n^{2c} is an integer from 1 to 16;
X^{2c} is a bond or -(OCH₂CH₂)n^{4c}-, wherein n⁴ is an integer from 1 to 16;
X^{3c} is a bond, -CH₂-, or phenyl;
ring A is an optionally substituted 8-membered carbocycle or an optionally substituted 8-membered heterocycle;
R¹ is an optional alkyl or aryl substituent;
X^{4c} is a bond, O, -C(O)- or a carbon atom which forms a cyclopropyl group with two carbons of ring A;
n^{3c} is an integer from 1 to 5;
X^{5c} is a bond or -(OCH₂CH₂)n^{5c}-, wherein n^{5c} is an integer from 1 to 16;
X^{6c} is a bond or -(CH₂)n^{6c}-, wherein n^{6c} is an integer from 1 to 5; and
Y² is an ester group, an amide group, or a succinimide group or a ring-opened product thereof, each of which is covalently bound to the peptide compound.

According to one preferred embodiment, linker A has a structure according to Formula (LA-VII) or a salt thereof or a [2+3]-cycloaddition regioisomer thereof: 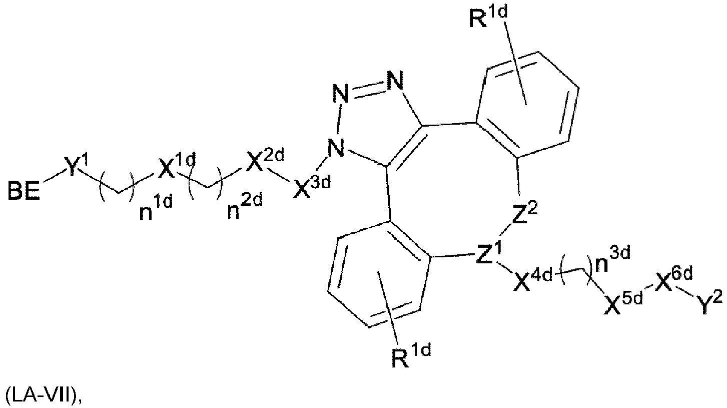 wherein
BE indicates attachment of linker A to the solid base element;
Y¹ is S or -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element;
n^{1d} is an integer from 1 to 6, preferably 2 to 4 (e.g. 3);
X^{1d} is a bond, -C(O)NH- or-NHC(O)-;
n^{2d} is an integer from 1 to 16;
X^{2d} is a bond or -(OCH₂CH₂)n^{4d}-, wherein n^{4d} is an integer from 1 to 16;
X^{3d} is a bond, -CH₂-;
R^{1d} is one to four optional substituents, and preferably R^{1d} is absent;
Z¹ is N, O or CH,
Z² is CH₂, CH, or C(O),
X^{4d} is a bond, O, -C(O)- or a carbon atom which forms a cyclopropyl group with Z¹ and Z²;
n^{3d} is an integer from 1 to 5;
X^{5d} is a bond or -(OCH₂CH₂)n^{5d}-, wherein n^{5d} is an integer from 1 to 16;
X^{6d} is a bond or -(CH₂)n^{6d}-, wherein n^{6d} is an integer from 1 to 5; and 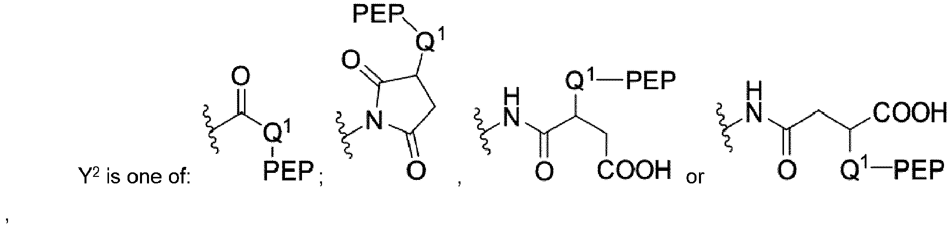
wherein Q¹ is NH, O or S of an amino acid of the peptide compound and PEP is the peptide compound.

According to one embodiment, the linker A has a structure according to Formula (LA-VII) or a salt thereof or a [2+3]-cycloaddition regioisomer thereof: 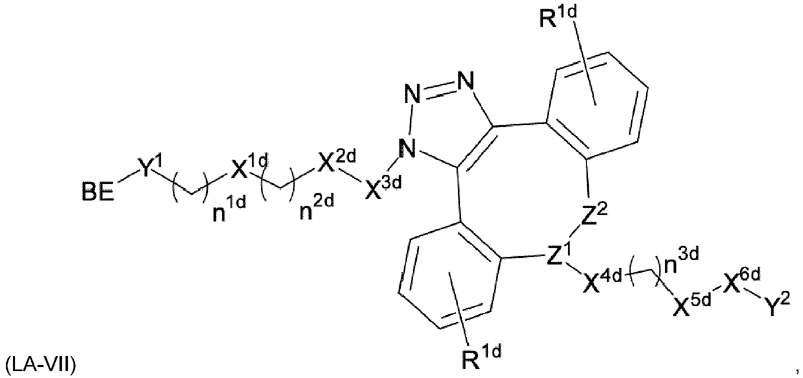 wherein
BE indicates attachment of linker A to the solid base element;
Y¹ is -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element
n^{1d} is an integer from 1 to 6, preferably 2 to 4 (e.g. 3);
X^{1d} is -C(O)NH- or -NHC(O)-;
n^{2d} is an integer from 1 to 16, preferably 2 to 4 (e.g. 2);
X^{2d} is -(OCH₂CH₂)n^{4d}-, wherein n^{4d} is an integer from 1 to 16;
X^{3d} is a bond or -CH₂-;
R^{1d} is one to four optional substituents, and preferably R^{1d} is absent;
Z¹ is N, O or CH;
Z² is CH₂, CH, or C(O);
X^{4d} is a bond, O, -C(O)- or a carbon atom which forms a cyclopropyl group with Z¹ and Z²;
n^{3d} is an integer from 1 to 5;
X^{5d} is a bond or -(OCH₂CH₂)n^{5d}-, wherein n^{5d} is an integer from 1 to 16;
X^{6d} is a bond or -(CH₂)n^{6d}-, wherein n^{6d} is an integer from 1 to 5; and
Y² is one of: 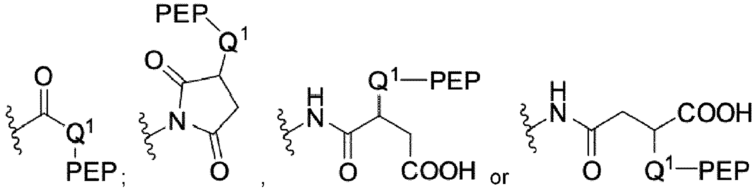 ,
wherein Q¹ is NH, O or S of an amino acid of the peptide compound and PEP is the peptide compound.

According to one embodiment, linker A has a structure according to Formula (LA-VII) or a salt thereof or a [2+3]-cycloaddition regioisomer thereof: 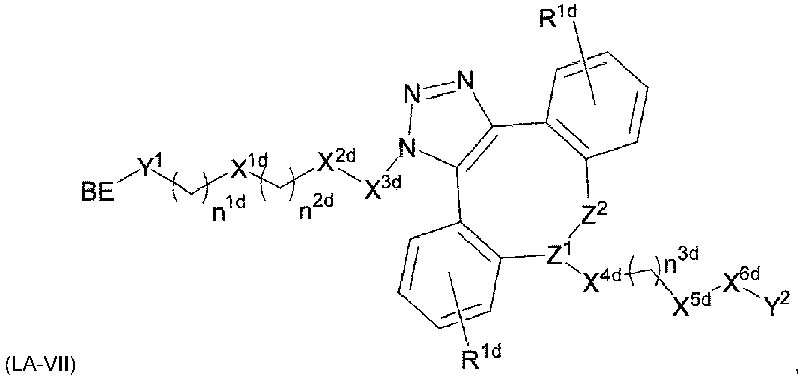 wherein
BE indicates attachment of linker A to the solid base element;
Y¹ is -O-Si(R²)-, wherein R² is each independently selected from OH, alkyl, alkoxy, OBE, and -O-Si^{x}, wherein Si^{x} is a silicon atom of another linker A or a silicon atom of an optional additional silane compound being attached to the surface of the solid base element
n^{1d} is an integer from 2 to 4 (e.g. 3);
X^{1d} is -NHC(O)-;
n^{2d} is an integer from 2 to 4 (e.g. 2);
X^{2d} is -(OCH₂CH₂)n^{4d}-, wherein n^{4d} is an integer from 1 to 16;
X^{3d} is a bond;
R^{1d} is absent;
Z¹ is N;
Z² is CH₂;
X^{4d} is -C(O)-;
n^{3d} is an integer from 1 to 5 (e.g. 2);
X^{5d} is a bond;
X^{6d} is a bond; and
Y² is one of: 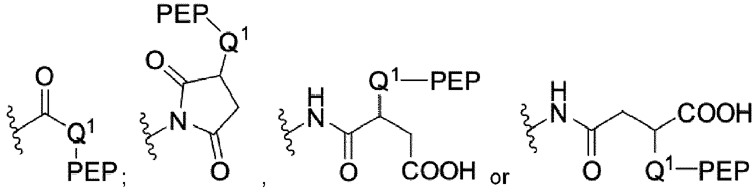 ,
wherein Q¹ is NH, O or S of an amino acid of the peptide compound and PEP is the peptide compound.

According to one more specific embodiment, linker A has a structure according to Formula (LA-VII) or a salt thereof or a [2+3]-cycloaddition regioisomer thereof: 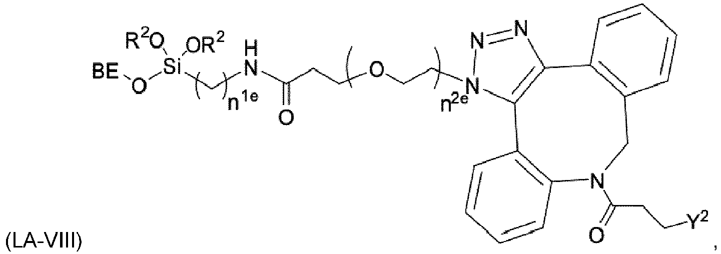
BE indicates attachment of linker A to the solid base element;
R² is each independently selected from OH, alkyl, alkoxy, OBE, and -O-Si^{x}, wherein Si^{x} is a silicon atom of another linker A or a silicon atom of an optional additional silane compound being attached to the surface of the solid base element
n^{1e} is an integer from 2 to 4, optionally 3;
n^{2e} is an integer from 3 to 8, optionally 5; and
Y² is one of: 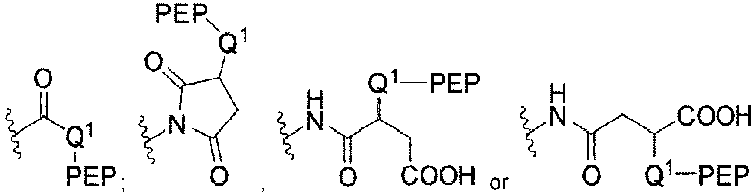 ,
wherein Q¹ is NH, O or S of an amino acid of the peptide compound and PEP is the peptide compound.

According to one embodiment, linker A has a structure according to Formula (LA-VII) or a salt thereof or a [2+3]-cycloaddition regioisomer thereof: 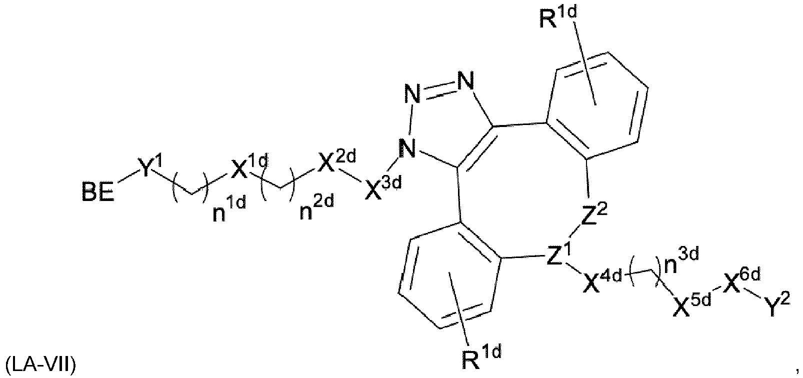 wherein
BE indicates attachment of linker A to the solid base element;
Y¹ is S;
n^{1d} is an integer from 1 to 6
X^{1d} is a bond
n^{2d} is an integer from 1 to 16;
X^{2d} is a bond or -(OCH₂CH₂)n^{4d}-, wherein n^{4d} is an integer from 1 to 16;
X^{3d} is a bond, -CH₂-;
R^{1d} is one to four optional substituents, and preferably R^{1d} is absent;
Z^{1d} is N, O or CH,
Z^{2d} is CH₂, CH, or C(O),
X^{4d} is a bond, O, -C(O)- or a carbon atom which forms a cyclopropyl group with Z¹ and Z²;
n^{3d} is an integer from 1 to 5;
X^{5d} is a bond or -(OCH₂CH₂)n^{5d}-, wherein n^{5d} is an integer from 1 to 16;
X^{6d} is a bond or -(CH₂)n^{6d}-, wherein n^{6d} is an integer from 1 to 5; and
Y² is one of: 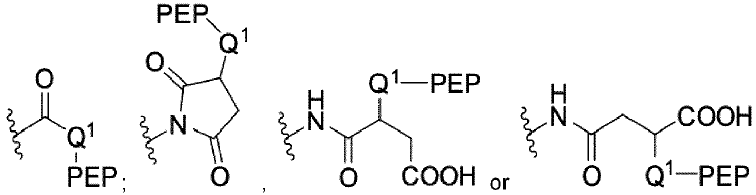
wherein Q¹ is NH, O or S of an amino acid of the peptide compound and PEP is the peptide compound.

According to one embodiment, linker A has a structure according to Formula (LA-IX) or a salt thereof or a [2+3]-cycloaddition regioisomer thereof: 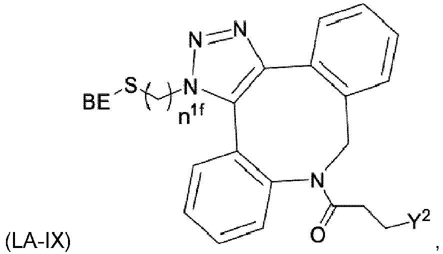 wherein
BE indicates attachment of linker A to the solid base element;
n^{1f} is an integer from 1 to 16, optionally 11,
and
Y² is one of: 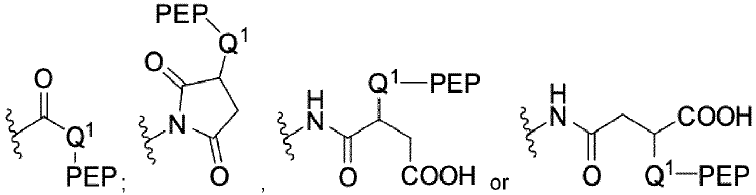
, wherein Q¹ is NH, O or S of an amino acid of the peptide compound and PEP is the peptide compound.

According to one embodiment, linker A has a structure according to Formula (LA-X) or a salt thereof or a [2+3]-cycloaddition regioisomer thereof: 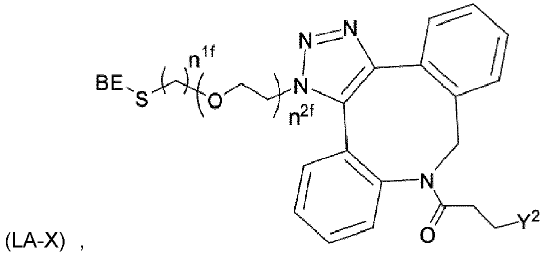 wherein
BE indicates attachment of linker A to the solid base element;
n^{1f} is an integer from 1 to 16, optionally 11,
n^{2f} is an integer from 1 to 16,
and
Y² is one of: 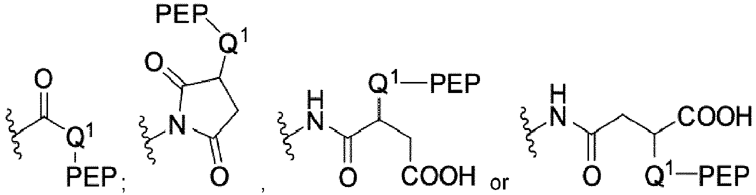
, wherein Q¹ is NH, O or S of an amino acid of the peptide compound and PEP is the peptide compound.

When linker A has a structure according to any one of Formula (LA-I) to (LA-VII) or a salt thereof or a or a [2+3]-cycloaddition regioisomer thereof, and wherein the Y¹ group is -Si(R²)-, then the solid base element is preferably a silicon crystal or a germanium crystal, preferably a silicon crystal, and more preferably is a silicon crystal wherein at least a part of the silicon crystal has a silicon dioxide surface, and wherein the Y¹ group is covalently bound to the silicon dioxide.

Alternatively, when linker A has a structure according to any one of Formula (LA-I) to (LA-VII) or a salt thereof or a or a [2+3]-cycloaddition regioisomer thereof, and wherein the Y¹ group is -S-, then the solid base element is preferably a germanium crystal.

According to one embodiment, linker A is obtainable or obtained by the steps a) to c) of the process according to the invention. According to one embodiment, linker A is obtainable or obtained by the step a), steps b1) to b4), and step c) of the process according to the invention.

Reference is made to the section below "Process according to the invention".

### Optional matrix compound

In addition to linker A, the first layer can further comprise a matrix compound, which is covalently bound to the solid base element.

The matrix compound may be a compound comprising a silicon-containing group which is covalently bound to the solid base element. The matrix compound is preferably a compound comprising a PEG group or mPEG group, and a silicon-containing group, wherein the silicon-containing group is covalently bound to the solid base element.

According to one embodiment, the matrix compound is a PEG-containing or mPEG-containing silane, and preferably a mPEG-containing silane. Additionally or alternatively, the matrix compound can be an optionally substituted alkyl sulfide, preferably an optionally substituted C1-C30-alkyl sulfide.

Hence, according to one embodiment, the matrix compound is:
(i) a PEG-containing or mPEG-containing silane, preferably a mPEG-containing silane, and/or
(ii) an optionally substituted alkyl sulfide, preferably an optionally substituted C1-C30 alkyl sulfide.

According to one embodiment, the first layer comprises a matrix compound, which is covalently bound to the solid base element,
wherein the matrix compound is a compound according to formula (MA-I) or a salt thereof:

(MA-I): BE-Y-X,

wherein
BE indicates attachment of the compound to the solid base element;
Y is S or -O-Si(R^{2g})-, wherein R^{2g} is each independently selected from OH, alkyl, alkoxy, OBE, and -O-Si^{y}, wherein Si^{y} is a silicon atom of a linker A or a silicon atom of another silane being attached to the surface of the solid base element, and
X is a group comprising a PEG group, mPEG group, or a alkyl group (e.g. a C1-C30 alkyl group).

According to one embodiment, the matrix compound is a compound according to formula (MA-II) or salt thereof: 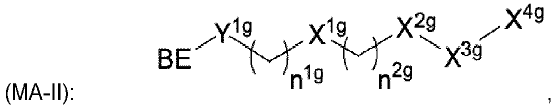 wherein
BE indicates attachment of the compound to the solid base element;
Y^{1g} is S or -O-Si(R^{2g})-, wherein R^{2g} is each independently selected from OH, alkyl, alkoxy, OBE, and -O-Si^{y}, wherein Si^{y} is a silicon atom of a linker A or a silicon atom of another silane being attached to the surface of the solid base element,
n^{1g} is an integer from 1 to 6;
X^{1g} is a bond, -C(O)NH- or-NHC(O)-;
n^{2g} is an integer from 1 to 15;
X^{2g} is a bond or -(OCH₂CH₂)n^{4g}-, wherein n^{4g} is an integer from 1 to 16;
X^{3g} is a bond, O or -CH₂-; and
X^{4g} is OH or CH₃.

The inventors found that the additional compound can function as a matrix on the surface of the base element which further reduces non-specific binding of media or sample components to the surface. Furthermore, the use of a matrix compound in combination with linker A allows for fine tuning the amount of linker A to be bound to the surface of the base element, and allows for spacing the linker A on the surface of the base element. Therefore, attaching the "matrix compound" to the surface of the base element such as a ATR waveguide (e.g. silicon crystal) in addition to linker A can improve the performance for detecting a biomarker.

According to one embodiment, the matrix compound is a compound according to formula (MA-II) or salt thereof: 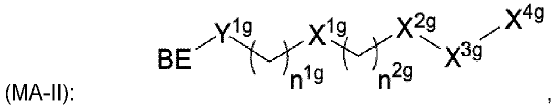 wherein
BE indicates attachment of the compound to the solid base element;
Y^{1g} is -O-Si(R^{2g})-, wherein R^{2g} is each independently selected from OH, alkyl, alkoxy, OBE, and -O-Si^{y}, wherein Si^{y} is a silicon atom of a linker A or a silicon atom of another silane being attached to the surface of the solid base element;
n^{1g} is an integer from 1 to 6, optionally 2 to 4 (e.g. 3);
X^{1g} is a -C(O)NH- or-NHC(O)-;
n^{2g} is an integer from 1 to 15, optionally 2 to 6 (e.g. 2);
X^{2g} is-(OCH₂CH₂)n^{4g}-, wherein n^{4g} is an integer from 1 to 16, optionally 1 to 5 (e.g. 2);
X^{3g} is a bond, O, or -CH₂-; and
X^{4g} is OH or CH₃.

According to one embodiment, the matrix compound is a compound according to formula (MA-II) or salt thereof: 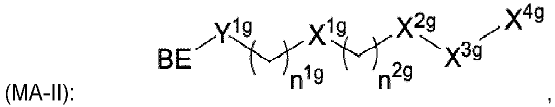 wherein
BE indicates attachment of the compound to the solid base element;
Y^{1g} is -O-Si(R^{2g})-, wherein R^{2g} is each independently selected from OH, alkyl, alkoxy, OBE, and -O-Si^{y}, wherein Si^{y} is a silicon atom of a linker A or a silicon atom of another silane being attached to the surface of the solid base element;
n^{1g} is an integer from 2 to 4 (e.g. 3);
X^{1g} is -NHC(O)-;
n^{2g} is an integer from 2 to 6 (e.g. 2);
X^{2g} is-(OCH₂CH₂)n^{4g}-, wherein n^{4g} is an integer from 1 to 5 (e.g. 2);
X^{3g} is a O; and
X^{4g} is CH₃.

According to one embodiment, the matrix compound is a compound according to formula (MA-II) or salt thereof: 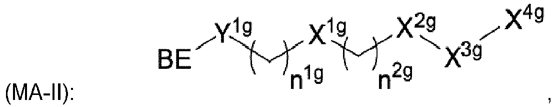 wherein
BE indicates attachment of the compound to the solid base element;
Y^{1g} is S;
n^{1g} is an integer from 1 to 6;
X^{1g} is a bond;
n^{2g} is an integer from 1 to 15;
X^{2g} is a bond;
X^{3g} is a bond; and
X^{4g} is CH₃.

When the compound according to Formula (MA-II) has a Y^{1g} group being -Si(R^{2g})-, then the solid base element is preferably a silicon crystal or a germanium crystal, preferably a silicon crystal, and more preferably is a silicon crystal wherein at least a part of the silicon crystal has a silicon dioxide surface, and wherein the Y^{1g} group is covalently bound to the silicon dioxide.

Alternatively, when the compound according to Formula (MA-II) has a Y^{1g} group being -S-, then the solid base element is preferably a germanium crystal.

### Second layer comprising a peptide compound

The molecular probe functionalized element according to the invention comprises a second layer comprising a peptide compound. The peptide compound is covalently bound to the solid base element via linker A and to the molecular probe via linker B. Preferably, the second layer has the function of a blocking layer.

The second layer can further comprise peptide compounds which are covalently bound to the solid base element via linker A but which are not attached to a molecular probe. In other words, the second layer can comprise peptide compounds which are not functionalized by a molecular probe via linker B, but which solely function as part of a blocking layer.

Hence, according to one preferred embodiment, the second layer comprises peptide compounds which are covalently bound to the solid base element via linker A but which are not attached to a molecular probe.

The second layer can comprise a single type of peptide compound or a mixture of peptide compounds. According to one embodiment, the second layer comprises or consists of a single type of peptide compound.

According to one preferred embodiment, the second layer comprises or consists of a mixture of peptide compounds. In case the second layer comprises or consists of a mixture of peptide compounds, different peptide compounds can be attached to the solid base element via linker A.

According to one preferred embodiment, the peptide compound of the second layer is selected from the group consisting of peptides, substituted peptides, proteins, protein fragments, and mixtures thereof. A substituted peptide can be, for example, a PEGylated peptide.

The peptide compound is preferably a peptide compound which has no or essentially no binding affinity for the target analyte of the molecular probe or a fluid containing said target analyte such as a body fluid (e.g. , intestinal fluid, ocular fluid, pulmonary fluid, blood or CSF). The peptide compound is preferably a peptide compound which is inert towards the target analyte of the molecular probe or a fluid containing the target analyte such as a body fluid (e.g. , intestinal fluid, ocular fluid, pulmonary fluid, blood or CSF).

The peptide compound may be an optionally substituted natural, semi-synthetic or synthetic peptide, optionally having an amino acid chain length between 2 to 600 amino acids, optionally 2 to 400 amino acids, optionally 2 to 200 amino acids, optionally 2 to 100 amino acids, and optionally 4 to 100 amino acids. The peptide compound may be a natural, semi-synthetic or a synthetic peptide having an amine acid chain length between 2 to 100 amino acids, and optionally 4 to 100 amino acids. Preferably, the peptide compound comprises one or more amino acids, which contain an H₂N-, HO- or HS-group in its/their side chain.

The peptide compound can be a fragment of a protein. The fragment of the protein can be obtainable or obtained by protein hydrolysis. Thus, the peptide compound may be obtainable or obtained from a protein hydrolysate. For example, the peptide compound can be obtainable or obtained from a hydrolysate of albumin, casein, BSA, serum proteins (e.g. animal serum such as swine, horse, or goat serum), milk proteins, or mixtures thereof. Preferably, the peptide compound is obtainable or obtained from a casein hydrolysate.

The peptide compound may be an optionally substituted peptide, optionally having an amino acid chain length between 2 to 600 amino acids, optionally 2 to 400 amino acids, optionally 2 to 200 amino acids (e.g. 2 to 100 or 4 to 100), wherein the peptide is obtainable or obtained from a protein hydrolysate. The peptide compound may be an optionally substituted peptide having an amino acid chain length between 2 to 600 amino acids, optionally 2 to 400 amino acids, optionally 2 to 200 amino acids (e.g. 2 to 100 or 4 to 100), wherein the peptide is obtainable or obtained from a protein hydrolysate, wherein the protein is selected from the group of albumin, casein, BSA, serum proteins, milk proteins, and mixtures thereof. Preferably, the peptide compound is an optionally substituted peptide having an amino acid chain length between 2 to 100 amino acids, wherein the peptide is obtainable or obtained from a casein hydrolysate. The casein hydrolysate may be a hydrolysate of high purity casein. A suitable casein hydrolysate is the commercially available *"The blocking solution"* from Candor Bioscience GmbH.

According to one embodiment, the second layer comprises a mixture of peptide compounds, wherein the mixture of peptide compounds is a mixture of peptides which are obtainable or obtained from a protein hydrolysate, and wherein the peptides are optionally substituted, e.g. PEGylated.

According to one embodiment, the second layer comprises a mixture of peptide compounds, wherein the mixture of peptide compounds is a mixture of peptides which are obtainable or obtained from a casein hydrolysate, and wherein the peptides are optionally substituted, e.g. PEGylated.

### Third layer comprising a linker B

The molecular probe functionalized element according to the invention comprises a third layer comprising a linker B. Linker B is covalently bound to the peptide compound and to the molecular probe. Linker B is not particularly limited in terms of its chemical structure as long as linker B is suitable for covalently binding a peptide compound as defined herein to a molecular probe.

According to one embodiment, linker B comprises a peptide attachment group which is covalently bound to the peptide compound, and a molecular probe attachment group which is covalently bound to the molecular probe, and wherein the peptide attachment group is covalently bound to the molecular probe attachment group by a linking moiety, wherein the linking moiety comprises a group which is obtainable by a bioorthogonal ligation reaction.

Preferably, the linking moiety comprises an optionally substituted N-heterocyclic group, wherein the N-heterocyclic group is optionally selected from triazoles (e.g. 1,2,3-triazoles), dihydropyridazines, and pyridazines.

Preferably, the linking moiety comprises an optionally substituted triazole group which is obtainable or obtained by a [2+3]-cycloaddition between an azide compound and an alkyne compound ("Click ligation") or the linking moiety comprises an optionally substituted dihydropyridazine which is obtainable or obtained by a [2+4]-cycloaddition between an tetrazine compound and a trans-alkene compound ("Click ligation").

According to one embodiment, linker B comprises
a peptide attachment group which is covalently bound to the peptide compound,
a molecular probe attachment group which is covalently bound to the molecular probe,
wherein the peptide attachment group is covalently bound to the molecular probe attachment group via a linking moiety;
wherein the linking moiety comprises an optionally substitiuted N-heterocyclic group selected from triazoles, dihydropyridazines and pyridazines, which is optionally obtainable or obtained by a cycloaddition reaction, and
wherein the peptide attachment group is an ester group, an amide group, or a succinimide group or a ring-opened product thereof.

According to one embodiment, linker B has a structure according to any one of formula (LB-I) to (LB-VII) or a salt thereof: 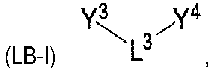 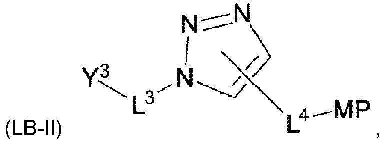 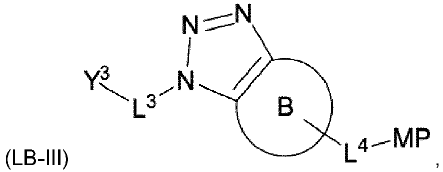 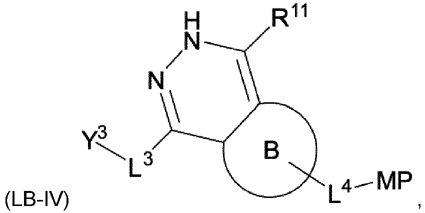 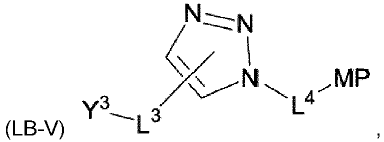 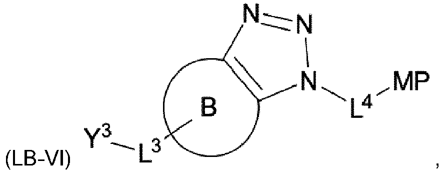 and 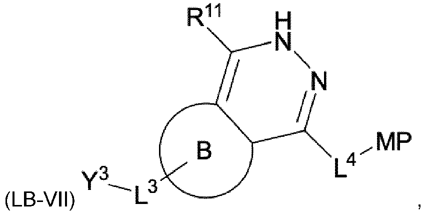 wherein
Y³ is an ester group, an amide group, or a succinimide group or a ring-opened product thereof, each of which is covalently bound to the peptide compound, and preferably Y³ is one of: 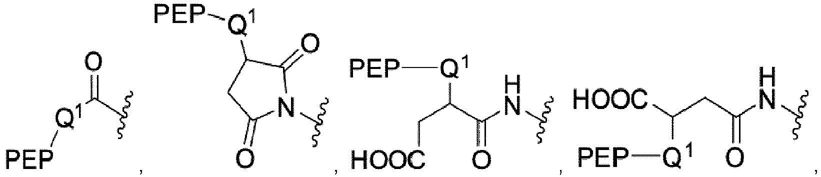 or a salt thereof, wherein Q¹ is NH, O or S of an amino acid of the peptide compound,
   PEP is the peptide compound,
L³ and L⁴, if present, are each a linking moiety,
Y⁴ is an ester group, an amide group, or a succinimide group or a ring-opened product thereof, each of which is covalently bound to the molecular probe,
MP is the molecular probe,
ring B, if present, is an optionally substituted 8-membered carbocycle or an optionally substituted 8-membered heterocycle;
R¹¹, if present, is H, optionally substituted alkyl or optionally substituted aryl.

According to one embodiment, linker B has a structure according to any one of formula (LB-VIII) to (LB-XIV) or a salt thereof for a : 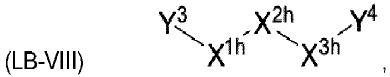 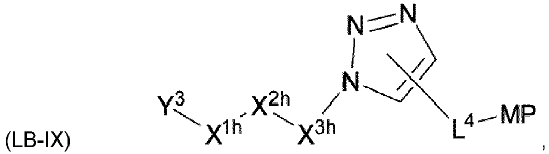 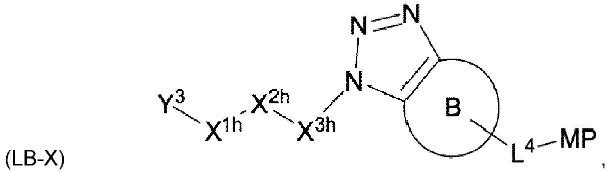 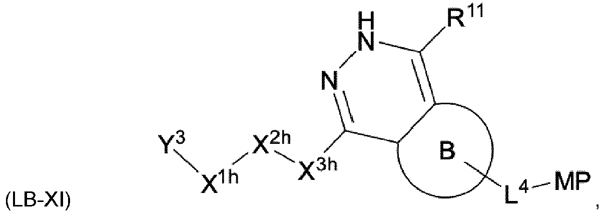 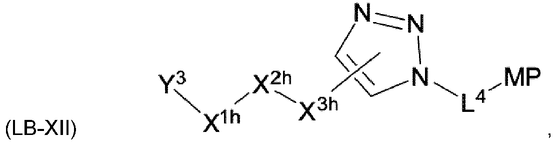 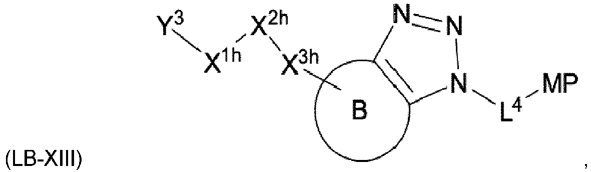 and 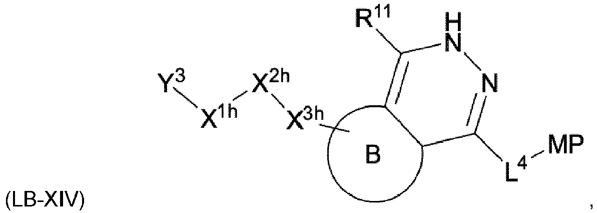 wherein
Y³ is an ester group, an amide group, or a succinimide group or a ring-opened product thereof, each of which is covalently bound to the peptide compound, and preferably Y³ is one of: 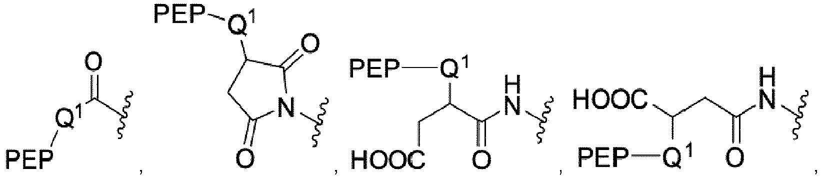 or a salt thereof, wherein Q¹ is NH, O or S of an amino acid of the peptide compound,
   PEP is the peptide compound,
L³ and L⁴, if present, are each a linking moiety,
Y⁴ is an ester group, an amide group, or a succinimide group or a ring-opened product thereof, each of which is covalently bound to the molecular probe,
MP is the molecular probe,
ring B, if present, is an optionally substituted 8-membered carbocycle or an optionally substituted 8-membered heterocycle;
R¹¹, if present, is H, optionally substituted alkyl or optionally substituted aryl;
X^{1h} is a bond, or (CH₂)n^{1h}, wherein n^{1h} is an integer between 1 to 10,
X^{2h} is a bond, or (OCH₂CH₂)n^{2h}, wherein n^{2h} is an integer between 1 to 15,
X^{3h} is a bond, C(O), or (CH₂)n^{3h}, wherein n^{3h} is an integer between 1 to 10.

According to one embodiment, linker B has a structure according to formula (LB-X) or (LB-XIII) or a salt thereof: 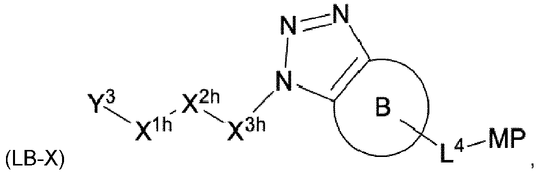 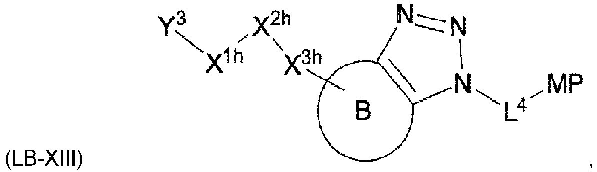 wherein
Y³ is an ester group, an amide group, or a succinimide group or a ring-opened product thereof, each of which is covalently bound to the peptide compound, and preferably Y³ is one of: 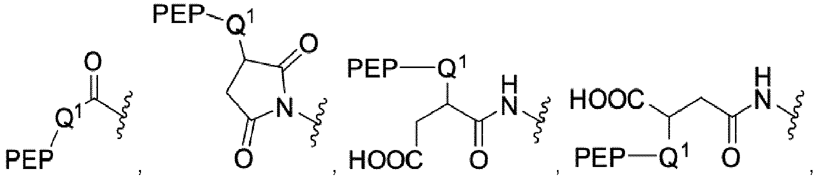 or a salt thereof, wherein Q¹ is NH, O or S of an amino acid of the peptide compound,
   PEP is the peptide compound,
L⁴ is a linking moiety,
MP is the molecular probe,
ring B is an optionally substituted 8-membered carbocycle or an optionally substituted 8-membered heterocycle;
X^{1h} is a bond, or (CH₂)n^{1h}, wherein n^{1h} is an integer between 1 to 10,
X^{2h} is a bond, or (OCH₂CH₂)n^{2h}, wherein n^{2h} is an integer between 1 to 15,
X^{3h} is a bond, C(O), or (CH₂)n^{3h}, wherein n^{3h} is an integer between 1 to 10.

According to one embodiment, linker B has a structure according to formula (LB-X)or a salt thereof: 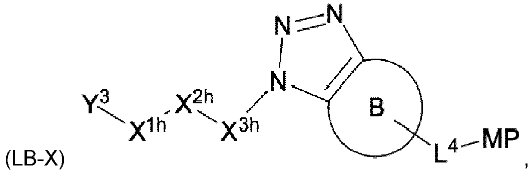 wherein
Y³ is an ester group, an amide group, or a succinimide group or a ring-opened product thereof, each of which is covalently bound to the peptide compound, and preferably Y³ is one of: 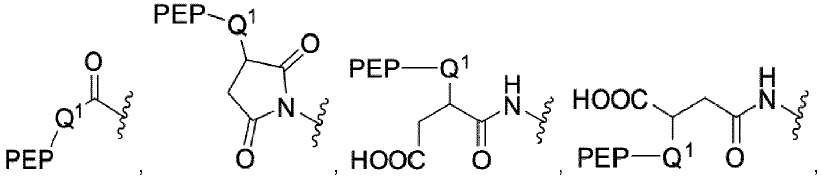 or a salt thereof, wherein Q¹ is NH, O or S of an amino acid of the peptide compound,
   PEP is the peptide compound,
L⁴ is a linking moiety,
MP is the molecular probe,
ring B is an optionally substituted 8-membered carbocycle or an optionally substituted 8-membered heterocycle;
X^{1h} is (CH₂)n^{1h}, wherein n^{1h} is an integer between 1 to 10, and preferably between 1 and 4,
X^{2h} is (OCH₂CH₂)n^{2h}, wherein n^{2h} is an integer between 1 to 15, and preferably between 2 to 10, and
X^{3h} is a bond.

According to one embodiment, linker B has a structure according to formula (LB-XIII) or a salt thereof: 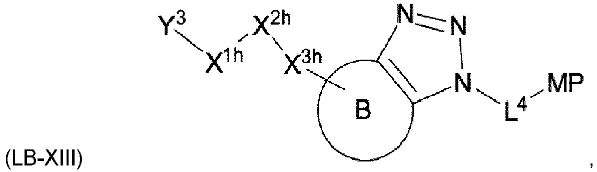 wherein
Y³ is an ester group, an amide group, or a succinimide group or a ring-opened product thereof, each of which is covalently bound to the peptide compound, and preferably Y³ is one of: 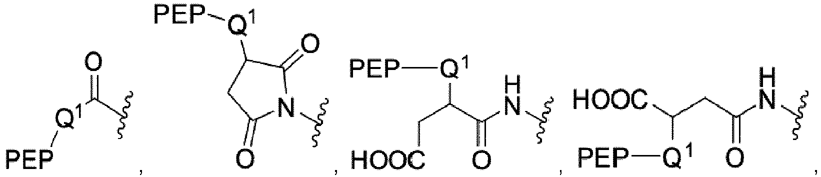 or a salt thereof, wherein Q¹ is NH, O or S of an amino acid of the peptide compound,
   PEP is the peptide compound,
L⁴ is a linking moiety,
MP is the molecular probe,
ring B is an optionally substituted 8-membered carbocycle or an optionally substituted 8-membered heterocycle;
X^{1h} is (CH₂)n^{1h}, wherein n^{1h} is an integer between 1 to 10,
X^{2h} is a bond, and
X^{3h} is C(O).

According to one embodiment, linker B has a structure to formula (LB-XV), or a salt thereof or a [2+3] cycloaddition regioisomer thereof: 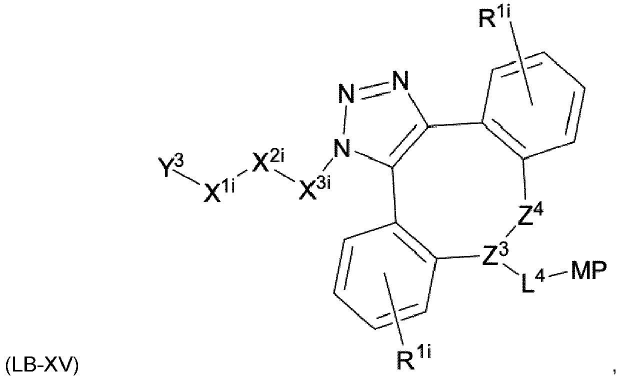 wherein
Y³ is an ester group, an amide group, or a succinimide group or a ring-opened product thereof, each of which is covalently bound to the peptide compound, and preferably Y³ is one of: 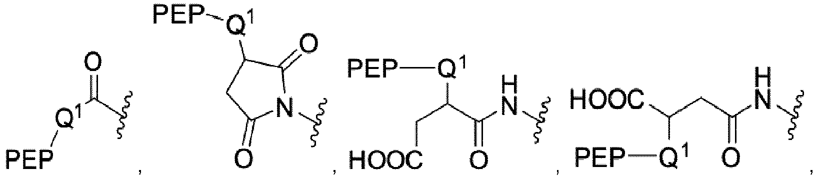 or a salt thereof, wherein Q¹ is NH, O or S of an amino acid of the peptide compound,
   PEP is the peptide compound,
L⁴ is a linking moiety,
MP is the molecular probe,
X¹ⁱ is a bond, or (CH₂)n¹ⁱ, wherein n¹ⁱ is an integer between 1 to 10, preferably is (CH₂)n¹ⁱ, wherein n¹ⁱ is an integer between 1 to 4,
X²ⁱ is a bond, or (OCH₂CH₂)n²ⁱ, wherein n²ⁱ is an integer between 1 to 15, and preferably is (OCH₂CH₂)n²ⁱ, wherein n²ⁱ is an integer between 2 to 10,
X³ⁱ is a bond or (CH₂)n³ⁱ, wherein n³ⁱ is an integer between 1 to 10, preferably is a bond,
R¹ⁱ is from one to four optional substituents, and preferably R¹ⁱ is absent,
Z³ is N or CH, preferably N.
Z⁴ is CH₂.

According to one embodiment, linker B has a structure to formula (LB-XVI), or a salt thereof or a [2+3] cycloaddition regioisomer thereof: 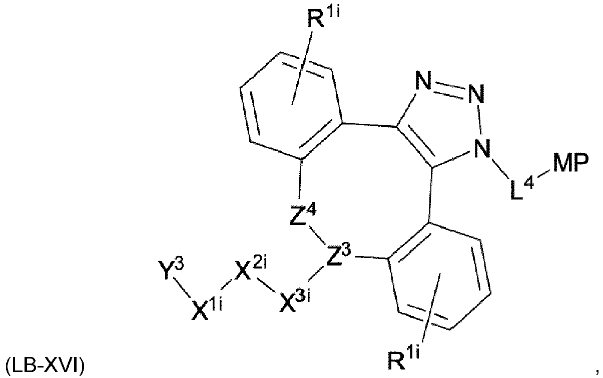 wherein
Y³ is an ester group, an amide group, or a succinimide group or a ring-opened product thereof, each of which is covalently bound to the peptide compound, and preferably Y³ is one of: 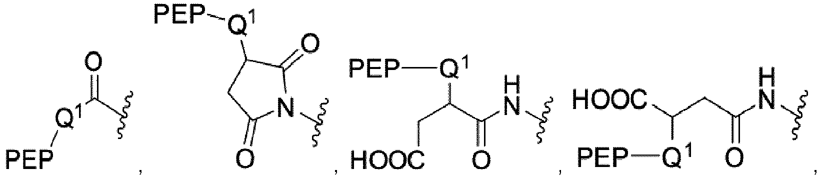 or a salt thereof, wherein Q¹ is NH, O or S of an amino acid of the peptide compound,
   PEP is the peptide compound,
L⁴ is a linking moiety, and preferably a linking moiety comprising (i) a PEG linker, and (ii) an ester group, an amide group, or a succinimide group or a ring-opened product thereof, each of which is covalently bound to the molecular probe,
MP is the molecular probe,
X¹ⁱ is a bond, or (CH₂)n¹ⁱ, wherein n¹ⁱ is an integer between 1 to 10, preferably is (CH₂)n¹ⁱ, wherein n¹ⁱ is an integer between 1 to 10,
X²ⁱ is a bond, or (OCH₂CH₂)n²ⁱ, wherein n²ⁱ is an integer between 1 to 15, and preferably is a bond,
X³ⁱ is C(O),
R¹ⁱ is from one to four optional substituents, and preferably R¹ⁱ is absent,
Z³ is N or CH, preferably N.
Z⁴ is CH₂.

According to one embodiment, linker B has a structure to formula (LB-XV), or a salt thereof or a [2+3] cycloaddition regioisomer thereof: 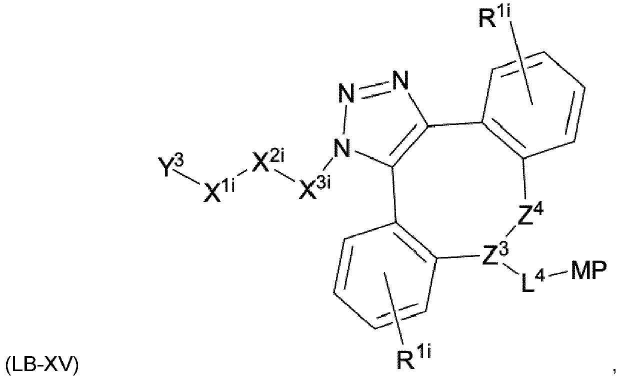 wherein
Y³ is an ester group, an amide group, or a succinimide group or a ring-opened product thereof, each of which is covalently bound to the peptide compound, and preferably Y³ is : 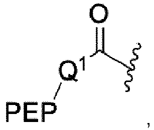 wherein Q¹ is NH of an amino acid of the peptide compound,
   PEP is the peptide compound,
L⁴ is a linking moiety, and preferably a linking moiety comprising (i) a PEG linker, and (ii) an ester group, an amide group, or a succinimide group or a ring-opened product thereof, each of which is covalently bound to the molecular probe,
MP is the molecular probe,
X¹ⁱ is a bond, or (CH₂)n¹ⁱ, wherein n¹ⁱ is an integer between 1 to 10, preferably is (CH₂)n¹ⁱ, wherein n¹ⁱ is an integer between 1 to 4 (e.g. 2),
X²ⁱ is a bond, or (OCH₂CH₂)n²ⁱ, wherein n²ⁱ is an integer between 1 to 15, and preferably is (OCH₂CH₂)n²ⁱ, wherein n²ⁱ is an integer between 2 to 10 (e.g. 7),
X³ⁱ is a bond or (CH₂)n³ⁱ, wherein n³ⁱ is an integer between 1 to 10, preferably is a bond,
R¹ⁱ is from one to four optional substituents, and preferably R¹ⁱ is absent,
Z³ is N or CH, preferably N.
Z⁴ is CH₂.

### Fourth layer comprising a molecular probe

The molecular probe functionalized element comprises a fourth layer comprising a molecular probe. The molecular probe is covalently bound to the peptide compound by linker B.

The molecular probe is not particularly limited and may be selected by the person of skill according to the target analyte which is to be detected and/or analyzed, and/or the target chemical interaction which is to be detected or analyzed. The molecular probe functionalized element can also comprise two or more different molecular probes, such as antibodies or the like.

According to one embodiment, the molecular probe is an antigen, an antibody, a fragment of an antibody, an antibody fusion protein or a fusion protein with an antibody fragment, an antibody conjugate or a conjugate with an antibody fragment, a protein complex which optionally contains an antibody fragment or a fusion protein thereof, an anticalin, a nanobody, a small organic molecule, a drug, a nucleic acid, an aptamer, a lipid, a carbohydrate, a peptide, or mixtures thereof.

According to one embodiment, the molecular probe is an antigen-binding protein, optionally selected from an antibody, a fragment of an antibody, an antibody fusion protein or a fusion protein with an antibody fragment, an antibody conjugate or a conjugate with an antibody fragment, or a protein complex which optionally contains an antibody fragment or a fusion protein thereof, an anticalin, a nanobody, and mixtures thereof.

According to one embodiment, the molecular probe is an anti-amyloid β antibody, an anti-alpha synuclein antibody, an anti-tau antibody, an anti-TDP-43 antibody, or a fragment, a fusion protein and/or a conjugate of said antibodies. According to one embodiment, the molecular probe is an anti-amyloid β antibody, an anti-alpha synuclein antibody, an anti-tau antibody, an anti-TDP-43 antibody, or a fragment, a fusion protein and/or a conjugate of said antibodies and/or mixtures of said antibodies. The anti-Amyloid β antibody may be selected from the following list: 2E9, H31L21, 11A50-B10, 12B2, 4G8, 11H3, MOAB-2, anti Aβ 25-35, A8978, 5C3, 8G7, 6G12, 1E8, and 32A1. The anti-alpha synuclein antibody may be 4B12, AKS5946,S5566. The anti-tau antibody may be tau-5 or tau396. The anti-TDP-43 antibody may be 1HCLC.

According to one embodiment, the molecular probe is an anti-amyloid β antibody, or a fragment, a fusion protein, and/or an conjugate thereof.

According to one embodiment, the molecular probe is an anti-alpha synuclein antibody, or a fragment, a fusion protein, and/or an conjugate thereof.

According to one embodiment, the molecular probe is capable of forming a complex with a protein which is associated with or may cause a proteopathy. Proteopathy is a class of disease in which one or more proteins are misfolded and/or become structurally abnormal, which can disrupt the function of cells, tissues, and/or organs. Proteopathies and proteins associated with proteopathies are known to the skilled person and include, but are not limited to, amyloid beta-peptide, tau proteins, alpha synuclein, TDP-43, and Huntingtin.

According to one embodiment, the molecular probe is capable of forming a complex with a target analyte selected from the group consisting of amyloid-beta (Aβ) peptides and isoforms thereof, alpha synuclein, tau protein, TDP-43, human islet amyloid polypeptide (hIAPP), Prion protein, and p53, and optionally selected from the group consisting of amyloid-beta (Aβ) peptides and isoforms thereof, alpha synuclein, tau protein and TDP-43. The amyloid-beta (Aβ) peptides may be monomeric, oligomeric or fibrillar amyloid-beta (Aβ) peptides.

### Device according to the invention

In one aspect, the present invention provides a device comprising a molecular probe functionalized element according to the invention.

Preferably, the device is a biosensor. Biosensors are well-known in the art. A biosensor is analytical device for the detection of a target analyte. A biosensor comprises a bioreceptor element (molecular probe) and a transduction element. The bioreceptor element (molecular probe) can interact with the target analyte to create a physical-chemical change, which is transformed into a measurable signal by the transduction element.

According to a preferred embodiment, the device is an optical biosensor. The optical biosensor may be based on surface plasmon resonance (SPR) microscopy, infrared ATR spectroscopy, Raman spectroscopy, colorimetric microscopy, fluorescent microscopy, luminescent microscopy, or combinations of these techniques. The optical biosensor may comprise a flow chamber for passing a sample flow over the molecular probe functionalized element according to the invention and a pump.

According to one preferred embodiment, the device is an SPR- or ATR-IR-based biosensor, and more preferably the device is a ATR-IR-based biosensor. Such devices are known in the art, and are for example described in Nabers et al, Anal. Chem. 2016, 88, 27552762.

### Process according to the invention

In one aspect, the present invention is a process for preparing a molecular probe functionalized element according to the invention. The process comprises the steps of:
a) providing a solid base element;
b) preparing a linker A precursor which is covalently bound to the surface of the solid base element;
c) reacting the linker A precursor with a peptide compound to covalently bind the peptide compound to the solid base element via linker A;
d) preparing a linker B precursor which is covalently bound the peptide compound; and
e) reacting the linker B precursor with a molecular probe to covalently bind the molecular probe to the peptide via linker B.

### Step a)

In step a), a solid base element is provided. As regards the definition of the solid base element it is referred to the definitions, the embodiments and preferred embodiments of the solid base element as defined herein for the molecular probe functionalized element according to the invention.

According to one embodiment, the solid base element is obtained by oxidizing at least a part of the surface of the IR-transparent optical element, and preferably of the ATR-IR waveguide (e.g. silicon crystal or germanium crystal). During the oxidative treatment (e.g. chemical oxidant or plasma treatment), at least a part of the surface of the is converted to an oxide layer which can improve attachment of linker compounds in subsequent step b).

According to one preferred embodiment, the solid base element is obtained by oxidizing at least a part of the surface of a silicon crystal, e.g. by a chemical oxidizing agent or plasma treatment. During the oxidative treatment, at least a part of the surface of the silicon crystal is converted to silicon dioxide. Before carrying out the plasma treatment, the surface of the silicon crystal may be polished.

### Step b)

In step b), a linker A precursor is prepared which is covalently bound to the surface of the solid base element.

Preferably, the linker A precursor comprises a peptide-reactive group, preferably an amine-reactive group (e.g. an active ester), which allows for covalently binding a peptide compound. "Peptide-reactive groups" are known in the art. Such groups are used to attach a compound to functional residues (e.g. amine acid residues) of a peptide such as amino groups (e.g. of a lysine residue), thiol (e.g. of cysteine), alcohol (e.g. of serine), and the like.

The linker A precursor may be prepared in a stepwise manner.

Preferably, the linker A precursor is prepared by first reacting the surface of the solid base element with a linker A1 to obtain a surface which is at least partially functionalized by a click-reactive group, and then reacting the at least partially functionalized surface with a linker A2 by a click ligation to obtain a linker A precursor which is covalently bound to the surface of the solid base element.

"Click-reactive groups" are well known in the art. Click-reactive groups are, but not limited to, alkynes, strained cycloalkynes (e.g. cyclooctynes wherein optionally one or more carbon atom of the ring is substituted by a heteroatom such as N), strained cycloalkenes (e.g. trans-cyclooctenes), azides, tetrazines, nitrones, and the like. Click-reactive groups can be reacted with one another in specific combinations ("Click reactions" or "Click ligations"), which are all well known to the person of skill in the art (e.g. azide with alkynes optionally under copper catalysis or azide with strained alkynes, etc).

According to one preferred embodiment, step b) comprises the steps of:
b1) providing a linker A1 comprising (i) a silyl group or a thiol group, and (ii) an azide group or an optionally substituted tetrazine group, preferably an azide group,
b2) optionally activating linker A1 provided in step b1),
b3) reacting the linker A1 provided in step b1) or the compound obtained in step b2) with the solid base element to obtain an at least partially functionalized surface,
b4) reacting the at least partially functionalized surface of the solid base element obtained in step b3) with a linker A2 comprising (i) an alkyne group or a trans-alkene group, (ii) and a peptide-reactive group selected from carboxylic acid, esters, active esters and maleimide,
to obtain a linker A precursor which is covalently attached to the surface of the base element.

The inventors surprisingly found that, functionalizing the surface of the solid base element (e.g. a waveguide for an optical biosensor such as a silicon crystal) with a click reactive group such as an azide or a tetrazine group, it is possible to obtain a stable and densely functionalized surface. The functionalized surface may be prepared and stored before using the same to provide the molecular probe functionalized element according to the invention. This is particularly advantageous for preparing shortly before use a molecular probe functionalized element which is highly reactive, which in turn improves the suitability of the molecular probe functionalized element according to one embodiment of the invention for an implementation in point-of-care (POC) testing devices.

In step b1), a linker A1 is preferably provided, wherein the linker A1 comprises (i) a silyl group or a thiol group, and (ii) an azide group or an optionally substituted tetrazine group, preferably an azide group.

According to one embodiment, linker A1 has a structure according to formula (LA1-I) or a salt thereof: 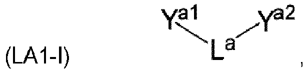 wherein
Y^{a1} is -SH or -SiZ₃, wherein each Z is independently selected from -O-(CH₂)x-CH₃, -(CH₂)y-CH₃, and halogen, wherein x is an integer between 0 to 4, and y is an integer between 0 to 6,
L^{a} is a linking moiety, and
Y^{2a} is -N₃ or 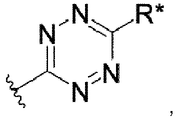 wherein R* is an optional alkyl substituent (e.g. methyl).

According to one embodiment, linker A1 has a structure according to formula (LA1-I) or a salt thereof:
(LA1-I) 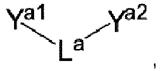
wherein
Y^{a1} is -SiZ₃, wherein each Z is independently selected from -O-(CH₂)x-CH₃, -(CH₂)y-CH₃, and halogen, wherein x is an integer between 0 to 4, and y is an integer between 0 to 6, and preferably wherein each Z is -OCH₃ or -OCH₂CH₃,
L^{a} is a linking moiety, and
Y^{a2} is -N₃ or 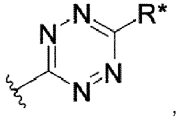 wherein R* is an optional alkyl substituent (e.g. methyl), and preferably wherein Y^{a2} is -N₃.

According to one embodiment, linker A1 has a structure according to formula (LA1-II) or a salt thereof: 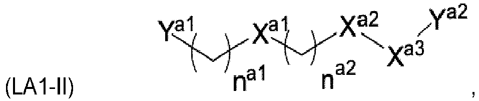 wherein
Y^{a1} is -SH or -SiZ₃, wherein each Z is independently selected from -O-(CH₂)x-CH₃, -(CH₂)y-CH₃, and halogen, wherein x is an integer between 0 to 4, and y is an integer between 0 to 6,
n^{a1} is an integer from 1 to 6;
x^{a1} is a bond, -C(O)NH- or-NHC(O)-;
n^{a2} is an integer from 1 to 16;
X^{a2} is a bond or -(OCH₂CH₂)z-, wherein z is an integer from 1 to 16;
X^{a3} is a bond or -CH₂-;
and
Y^{a2} is -N₃ or 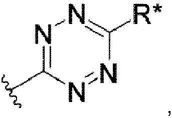 wherein R* is an optional alkyl substituent (e.g. methyl).

According to one embodiment, linker A1 has a structure according to formula (LA1-II) or a salt thereof: 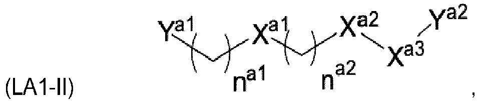 wherein
Y^{a1} is-SiZ₃, wherein each Z is independently selected from -O-(CH₂)x-CH₃, -(CH₂)y-CH₃, and halogen, wherein x is an integer between 0 to 4, and y is an integer between 0 to 6, and preferably wherein each Z is -OCH₃ or -OCH₂CH₃,
n^{a1} is an integer from 1 to 6;
X^{a1} is a bond, -C(O)NH- or -NHC(O)-;
n^{a2} is an integer from 1 to 16;
X^{a2} is a bond or -(OCH₂CH₂)z-, wherein z is an integer from 1 to 16;
X^{a3} is a bond or -CH₂-;
and
Y^{a2} is -N₃ or 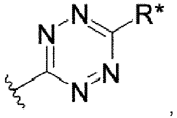 wherein R is an optional alkyl substituent (e.g. methyl), and preferably wherein Y^{a2} is -N₃.

In a specific embodiment, linker A1 has the following structure:

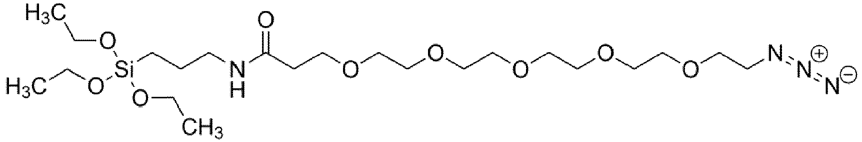

In optional step b2), linker A1 provided in b1) is activated. Activation step b2) may convert linker A1 in an intermediate, which is more reactive towards the surface of the solid base element. Specific conditions of activation step b2) depend on the chemical structure of linker A1 and can be chosen by the person of skill.

In step b3), the linker A1 provided in step b1) or the compound obtained in step b2) is reacted with the base element to obtain an at least partially functionalized surface. Preferably, linker A1 is selected to obtain an azide-functionalized surface in step b3).

In step b4), the at least partially functionalized surface of the solid base element obtained in step b3) is reacted with a linker A2 comprising (i) an alkyne group or a trans-alkene group, (ii) and a peptide-reactive group selected from carboxylic acid, carboxylic acid esters, active esters and maleimide,
to obtain a linker A precursor which is covalently attached to the surface of the solid base element.

The alkyne group (i) of linker A2 can be selected from optionally substituted 8-membered cycloalkynes, which optionally comprise heteroatoms in the 8-membered cycle. Such compounds are known to react with azides in strain-promoted click reaction. Strain promoted click reactions are reactions between a strained internal cyclic alkyne and an azide, and are known to the person of skill. The alkyne group (i) of linker A2 can be, but is not limited to, DBCO, DIBO, DIFO, or BCN. These abbreviations are well-known in the art. For illustration, DBCO is 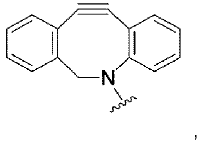 DIBO is 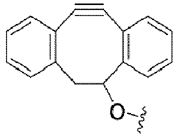 and DIFO is

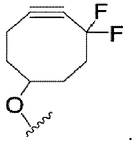

"Active esters" are well-known in the art, and can be reacted with an amino group to form an amide bond.

According to one embodiment, linker A2 has a structure according to formula (LA2-I) or a salt thereof: 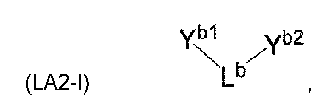 wherein Y^{b1} is 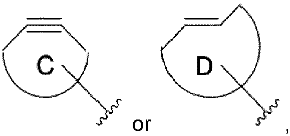 wherein ring C is an optionally substituted 8-membered cycloalkyne optionally comprising one or more heteroatoms, and ring D is an optionally substituted 8-membered cyclo-trans-alkene optionally comprising one or more heteroatoms,
L^{b} is a linking moiety, and
Y^{b2} is a peptide-reactive group selected from carboxylic acid, esters, active esters and N-linked maleimide, and optionally the peptide-reactive group Y^{b2} is selected from -COOH, -COOR, - COOR' and N-linked maleimide, wherein R is optionally substituted aryl or alkyl, more preferably optionally substituted C₆-aryl or C₁₋₆-alkyl, and R' is -NHS or -pentafluorophenyl (PFP).
"N-linked maleimide" means that the linker L is covalently bound to the nitrogen of the maleimide.

According to one embodiment, linker A2 has a structure according to formula (LA2-II) or a salt thereof: 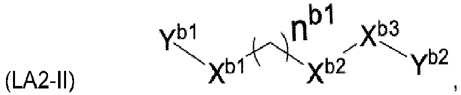 wherein Y^{b1} is 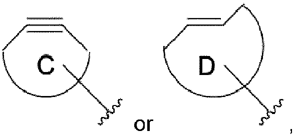 wherein ring C is an optionally substituted 8-membered cycloalkyne optionally comprising one or more heteroatoms, and ring D is an optionally substituted 8-membered cyclo-trans-alkene optionally comprising one or more heteroatoms,
X^{b1} is a bond, O, C(O), or a carbon atom which forms a cyclopropyl ring with two carbon atoms of ring A,
n^{b1} is an integer from 1 to 5, and
X^{b2} is a bond, O, or -(OCH₂CH₂)v-, wherein v is an integer from 1 to 15,
X^{b3} is a bond or (CH₂)w, wherein w is an integer from 1 to 5,
Y^{b2} is a peptide-reactive group selected from carboxylic acid, esters, active esters and N-linked maleimide, and optionally the peptide-reactive group Y^{b2} is selected from -COOH, -COOR, - COOR' and N-linked maleimide, wherein R is optionally substituted aryl or alkyl, more preferably optionally substituted C₆-aryl or C₁₋₆-alkyl, and R' is -NHS or -pentafluorophenyl (PFP).

According to one embodiment, linker A2 has a structure according to formula (LA2-II) or a salt thereof: 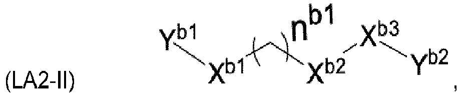 wherein Y^{b1} is 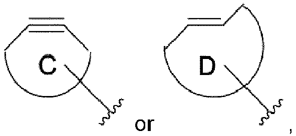 wherein ring C is an optionally substituted 8-membered cycloalkyne optionally comprising one or more heteroatoms, and ring D is an optionally substituted 8-membered cyclo-trans-alkene optionally comprising one or more heteroatoms, and optionally Y^{b1} is ring C which is 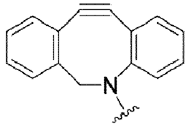 ,
X^{b1} is C(O),
n^{b1} is an integer from 1 to 5, and
X^{b2} is a bond or -(OCH₂CH₂)v-, wherein v is an integer from 1 to 15,
X^{b3} is a bond or (CH₂)w, wherein w is an integer from 1 to 5,
Y^{b2} is a peptide-reactive group selected from carboxylic acid, esters, active esters and N-linked maleimide, and optionally the peptide-reactive group Y^{b2} is selected from -COOH, -COOR, - COOR' and N-linked maleimide, wherein R is optionally substituted aryl or alkyl, more preferably optionally substituted C₆-aryl or C₁₋₆-alkyl, and R' is -NHS or -pentafluorophenyl (PFP).

According to one specific example, linker A2 has the following structure:

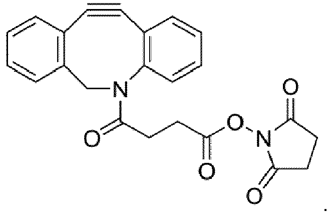

In step b4), a linker A precursor is preferably obtained which is covalently bound to the surface of the base element and which comprises a peptide-reactive group (e.g. an amine-reactive group such as an active ester). The reactivity of the peptide-reactive group can be exploited in subsequent step c) of the process according to the invention for covalently binding the peptide to the base element via a linker A, as defined herein above for the molecular probe functionalized element of the invention.

It is also possible to switch the click-reactive groups of linker A1 and linker A2. In such case, linker A1 can comprise the alkyne or trans-alkene group, and linker A2 can comprise the azide group or the optionally substituted tetrazine group. Thus, according to another embodiment, step b) comprises the steps of:
b1) providing a linker A1 comprising (i) a silyl group or a thiol group, and (ii) an alkyne group or a trans-alkene group,
b2) optionally activating linker A1 provided in b1),
b3) reacting the linker A1 provided in step b1) or the compound obtained in step b2) with the solid base element to obtain an at least partially functionalized surface,
b4) reacting the at least partially functionalized surface of the solid base element obtained in step b3) with a linker A2 comprising (i) an azide group or an optionally substituted tetrazine group, preferably an azide group, (ii) and a peptide-reactive group selected from carboxylic acid, esters, active esters and maleimide,
to obtain a linker A precursor which is covalently attached to the surface of the base element.

The process of the invention further comprises an optional step of reacting a matrix precursor compound with the solid base element to covalently attach a matrix compound to the surface of the base element. As regards the definition of the matrix compound, it is referred to the definition, the embodiments, and the preferred embodiments of the matrix compound as defined herein for the molecular probe functionalized element according to the invention.

This step can be carried before, during and/or after step b), and is preferably carried out during step b).

The step can be carried out simultaneously with step b1), optional step b2) and step b3). In case the step is carried out simultaneously with step b1), optional step b2) and step b3), the matrix precursor compound can be provided in form of a mixture with linker A1 in step b1). This mixture may then be used in optional step b2) and step b3 as defined above. The mixture may have a molar ratio of linker A1: matrix precursor compound of in the range of 1:99 to 99.9:0.1, optionally 25:75 to 99.9:0.1, optionally 50:50 to 99.9:0.1, optionally 75:25 to 99.9:0.1, optionally 90:10 to 99:1.

The matrix precursor compound may be a compound which comprises (i) a silyl group and (ii) a PEG group or mPEG group, or a compound which comprises (i) a thiol group and (ii) an optionally substituted alkyl group (e.g. an optionally substituted C1-C30 alkyl group).

The matrix precursor compound can have a structure according to formula (MA2-I) or a salt thereof: 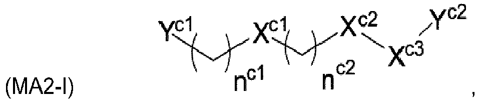 wherein
Y^{c1} is -SH or -SiZ₃, wherein each Z is independently selected from -O-(CH₂)x-CH₃, -(CH₂)y-CH₃, and halogen, wherein x is an integer between 0 to 4, and y is an integer between 0 to 6,
n^{c1} is an integer from 1 to 6;
x^{c1} is a bond, -C(O)NH- or-NHC(O)-;
n^{c2} is an integer from 1 to 16;
X^{c2} is a bond or -(OCH₂CH₂)r-, wherein r is an integer from 1 to 16;
X^{c3} is a bond, -CH₂-;
and Y^{c2} is OH, OCH₃, or CH₃.

The matrix precursor compound can have a structure according to formula (MA2-I) or a salt thereof: 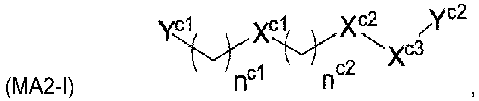 wherein
Y^{c1} is -SiZ₃, wherein each Z is independently selected from -O-(CH₂)x-CH₃, -(CH₂)y-CH₃, and halogen, wherein x is an integer between 0 to 4, and y is an integer between 0 to 6,
n^{c1} is an integer from 1 to 6;
X^{c1} is -C(O)NH- or-NHC(O)-;
n^{c2} is an integer from 1 to 16;
X^{c2} is -(OCH₂CH₂)r-, wherein r is an integer from 1 to 16;
X^{c3} is a bond, -CH₂-;
and Y^{c2} is OCH₃.

Specific embodiments of the matrix precursor compound have the following structures: 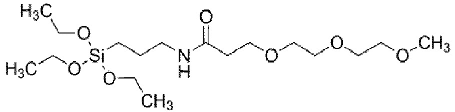 or

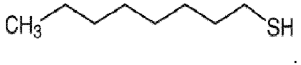

### Step c)

In step c), the linker A precursor is reacted with a peptide compound to covalently bind the peptide compound to the solid base element via linker A. As regards the definition of the peptide compound and the linker A, it is referred to the definitions, embodiments and preferred embodiments of the peptide compound and the linker A as defined herein for the molecular probe functionalized element of the invention.

The reaction of the peptide compound with the activated surface obtained in step b) or step b4) can block the surface of the solid base element. Thus, according to one embodiment, step c) is a blocking step.

The peptide compound may be provided in pure form or as part of a mixture such as a protein hydrolysate.

According to one embodiment, the peptide compound is provided as part of a protein hydrolysate, and optionally is part of a hydrolysate of albumin, casein, BSA, serum proteins (e.g. animal serum such as swine, horse, or goat serum), milk proteins, or mixtures thereof. Preferably, the peptide is provided in form a casein hydrolysate. The protein hydrolysate comprising the peptide compound can be contacted with the linker A precursor obtained in step b) or b4) to react the peptide compound with the linker A precursor. Preferably, the peptide compound is provided as part of a casein hydrolysate.

### Step d)

In step d), a linker B precursor is prepared which is covalently bound to the peptide compound.

Preferably, the linker B precursor comprises a click-reactive group, e.g. selected from alkynes, strained cycloalkynes (e.g. cyclooctynes wherein optionally one or more carbon atom of the ring is substituted by a heteroatom such as N), strained cycloalkenes (e.g. trans-cyclooctenes), azides, tetrazines, nitrones, and the like, which allows for covalently binding the molecular probe by a click ligation.

The linker B precursor may be prepared by reacting the peptide compound with a linker B1 as defined herein below.

According to one embodiment, linker B1 comprises (i) a peptide-reactive group (e.g. an amine-reactive group) selected from carboxylic acid, esters, active esters and maleimide, (ii) click-reactive group selected from alkynes, strained cycloalkynes, strained cycloalkenes, azides, and tetrazines.

According to one embodiment, linker B1 has a structure according to formula (LB1-I) or a salt thereof: 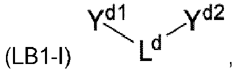 wherein
Y^{d1} is a peptide-reactive group selected from carboxylic acid, esters, active esters and N-linked maleimide, and optionally the peptide-reactive group Y^{d2} is selected from -COOH, -COOR, - COOR' and N-linked maleimide, wherein R is an optionally substituted aryl or alkyl, more preferably optionally substituted C₆-aryl or C₁₋₆-alkyl, and R' is -NHS or -pentafluorophenyl (PFP),
L^{d} is a linking moiety, optionally comprising a PEG group,
Y^{d2} is an optionally substituted click-reactive group selected from alkynes, strained cycloalkynes, strained cycloalkenes, azides, and tetrazines, and optionally is selected from -N₃, or 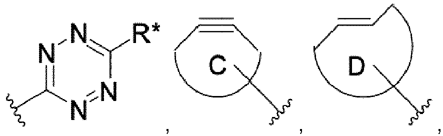 wherein R* is an optional alkyl or aryl substituent (e.g. methyl), wherein ring C is an optionally substituted 8-membered cycloalkyne optionally comprising one or more heteroatoms, and ring D is an optionally substituted 8-membered cyclo-trans-alkene optionally comprising one or more heteroatoms.

According to one embodiment, linker B1 has a structure according to formula (LB1-II) or a salt thereof: 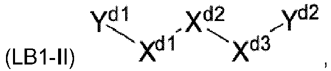 wherein
Y^{d1} is a peptide-reactive group selected from carboxylic acid, esters, active esters and N-linked maleimide, and optionally the peptide-reactive group Y^{d2} is selected from -COOH, -COOR, - COOR' and N-linked maleimide, wherein R is an optionally substituted aryl or alkyl, more preferably optionally substituted C₆-aryl or C₁₋₆-alkyl, and R' is -NHS or -pentafluorophenyl (PFP),
X^{d1} is a bond or (CH₂)s, wherein s is an integer from 1 to 10,
X^{d2} is a bond or (OCH₂CH₂)t, wherein t is an integer from 1 to 15,
X^{d3} is a bond, C(O), or (CH₂)q, wherein q is an integer from 1 to 10,
Y^{d2} is an optionally substituted click-reactive group selected from alkynes, strained cycloalkynes, strained cycloalkenes, azides, and tetrazines, and optionally is selected from -N₃, or 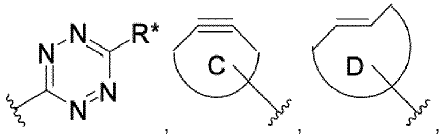 wherein R* is an optional alkyl or aryl substituent (e.g. methyl), wherein ring C is an optionally substituted 8-membered cycloalkyne optionally comprising one or more heteroatoms, and ring D is an optionally substituted 8-membered cyclo-trans-alkene optionally comprising one or more heteroatoms.

According to one embodiment, linker B1 has a structure according to formula (LB1-II) or a salt thereof: 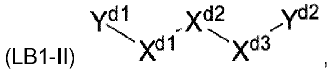 wherein
Y^{d1} is a peptide-reactive group selected from -COOH, -COOR, -COOR' and N-linked maleimide, wherein R is optionally substituted C₆-aryl or C₁₋₆-alkyl, and R' is -NHS orpentafluorophenyl (PFP),
X^{d1} is (CH₂)s, wherein s is an integer from 1 to 10,
X^{d2} is a bond or (OCH₂CH₂)t, wherein t is an integer from 1 to 15,
X^{d3} is a bond, C(O),
Y^{d2} is click-reactive group selected from -N₃, or 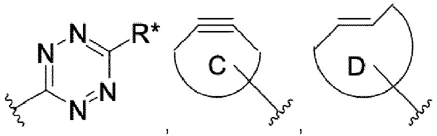 , wherein R* is an optional methyl, wherein ring C is an optionally substituted 8-membered cycloalkyne optionally comprising one or more heteroatoms, and ring D is an optionally substituted 8-membered cyclo-trans-alkene optionally comprising one or more heteroatoms.

According to one specific embodiment, linker B1 has one of the following structures:

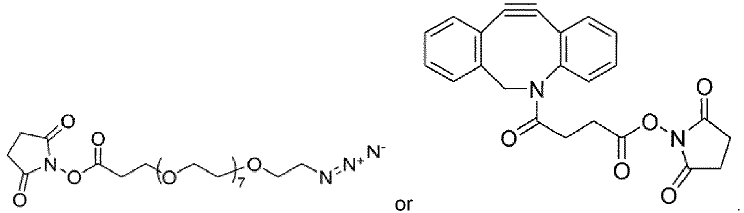

It may be that after step c) and/or step d), there are remaining reactive linker A precursors on the surface of the solid base element. In such case, it is possible, and sometimes preferred, to react the surface with a small molecule such as an amine compound (e.g. ethanol amine) to quench the remaining reactive linker A precursor after step c) and/or step d).

According to one embodiment, the process comprises the additional step of adding a quenching reagent between step c) and step d) and/or between step d) and step e). The quenching reagent may be any reagent which reacts with a peptide-reactive group (such as an active ester). For example, the quenching reagent may be an amine compound, e.g. ethanol amine.

It is possible, and sometimes preferred, to add the quenching reagent before reacting the molecular probe with the linker B precursor in step e). According to one embodiment, the process comprises the additional step of adding a quenching reagent between step d) and step e). According to one embodiment, the process comprises adding a quenching reagent (e.g. an amine compound such as ethanolamine) before step e).

### Step e)

In step e), the linker B precursor is reacted with a molecular probe to covalently bind the molecular probe to the peptide compound via linker B. As regards the definition of the molecular probe and linker B, it is referred to the definitions, embodiments and preferred embodiments of the molecular probe and the linker B as defined herein for the molecular probe functionalized element of the invention.

The molecular probe can be activated before reacting it with the linker B precursor. The molecular probe can be activated by attaching a click reactive group onto the molecular probe, which is suitable for a reaction with the click reactive group of the linker B precursor. A skilled person knows which combinations of click reactive groups react with each other in a click reaction.

According to one embodiment, the molecular probe is activated before step e) by reacting the molecular probe with a linker B2 comprising a click reactive group selected from selected from alkynes, strained cycloalkynes (e.g. cyclooctynes wherein optionally one or more carbon atom of the ring is substituted by a heteroatom such as N), strained cycloalkenes (e.g. trans-cyclooctenes), azides, and tetrazines.

According to one embodiment, the molecular probe is activated before step e) by reacting the molecular probe with a linker B2 comprising a click reactive group selected from strained cycloalkynes (e.g. cyclooctynes wherein optionally one or more carbon atom of the ring is substituted by a heteroatom such as N) and an azide. The molecular probe may be activated before step e) by reacting the molecular probe with an NHS-azide or NHS-DBCO. NHS-azide and NHS-DBCO can react with amines of the molecular probe, e.g. with a lysine residue of the molecular probe.

### Method according to the invention

In one aspect, the present invention provides a method for detecting a biomarker. The method comprises
a) bringing into contact the molecular probe functionalized element according to the invention with a sample suspected to comprise a target analyte;
b) detecting the biomarker based on an interaction between the molecular probe and the target analyte.

The sample may be a body fluid such as, but not limited to, intestinal fluid, ocular fluid, pulmonary fluid, blood or cerebrospinal fluid (CSF) sample. According to one embodiment, the sample is a blood or CSF sample. The sample may be brought into contact with the molecular probe functionalized element in a flow chamber.

According to one embodiment, the target analyte may be selected from the group consisting of amyloid-beta (Aβ) peptides, alpha synuclein, tau protein, TDP-43, human islet amyloid polypeptide (hIAPP), Prion protein, and p53, and optionally selected from the group consisting of amyloid-beta (Aβ) peptides and isoforms thereof, alpha synuclein, tau protein and TDP-43. The amyloid-beta (Aβ) peptides may be monomeric, oligomeric or fibrillar amyloid-beta (Aβ) peptides.

The interaction between the molecular probe and the target analyte may be detected by any suitable means detecting a biochemical interaction. The interaction may be detected by a spectroscopical method such as, but not limited to, infrared spectroscopy, UV/Vis spectroscopy, fluorescence spectroscopy, and the like.

According to one embodiment, the interaction between the molecular probe and the target analyte is detected by infrared spectroscopy, and preferably by ATR-IR spectroscopy.

According to one embodiment, the interaction between the molecular probe and the target analyte is detected by infrared spectroscopy, and preferably by ATR-IR spectroscopy, and wherein the biomarker is an amide band maximum of the target analyte. The amide band maximum may be an amide band maximum of an amyloid-beta peptide, and is optionally in the range of 1600 to 1700 cm⁻¹, optionally 1620 to 1670 cm⁻¹, and optionally 1640 to 1647 cm⁻¹.

Such methods are known in the art, and are for example described in Nabers et al, Anal. Chem. 2016, 88, 27552762.

### Use according to the invention

In one aspect, the present invention provides a use of molecular probe functionalized element according to the invention or of a device according to the invention for one or more of:
a companion diagnostic test,
diagnosing Alzheimer's disease, Parkinson's disease, amyotrophic lateral sclerosis, Diabetes, Huntington, Prion disease, or a tumor in a patient,
monitoring of therapy of patients with Alzheimer's disease, Parkinson's disease, amyotrophic lateral sclerosis , Diabetes, Huntington, Prion disease, or a tumor, and
screening of drugs for the treatment of Alzheimer's disease, Parkinson's disease, amyotrophic lateral sclerosis , Diabetes, Huntington, Prion disease, or a tumor.

The tumor is limited to a specific tumor but can be various types of tumors.

In one embodiment, the molecular probe functionalized element or the device is used for a companion diagnostic test.

In one embodiment, the molecular probe functionalized element or the device is used for diagnosing Alzheimer's disease, Parkinson's disease, amyotrophic lateral sclerosis, Diabetes, Huntington, Prion disease, or a tumor in a patient in a patient. In one embodiment, the molecular probe functionalized element or the device is used for monitoring of therapy of patients with Alzheimer's disease, Parkinson's disease, amyotrophic lateral sclerosis, Diabetes, Huntington, Prion disease, or a tumor in a patient. In one embodiment, the molecular probe functionalized element or the device is used for screening of drugs for the treatment of Alzheimer's disease, Parkinson's disease, amyotrophic lateral sclerosis, Diabetes, Huntington, Prion disease, or a tumor in a patient.

According to one embodiment, the molecular probe functionalized element or the device is used for diagnosing a protein misfolding disease in a patient and/or for monitoring of therapy of patients with a protein misfolding disease.

According to one embodiment, the molecular probe functionalized element or the device is used for diagnosing Alzheimer's disease and/or for monitoring of therapy of patients with Alzheimer's disease.

According to one embodiment, a molecular probe functionalized element according to the invention or of a device according to the invention is provided for use one or more of:
a companion diagnostic test,
diagnosing Alzheimer's disease, Parkinson's disease, amyotrophic lateral sclerosis, Diabetes, Huntington, Prion disease, or a tumor in a patient,
monitoring of therapy of patients with Alzheimer's disease, Parkinson's disease, amyotrophic lateral sclerosis , Diabetes, Huntington, Prion disease, or a tumor, and
screening of drugs for the treatment of Alzheimer's disease, Parkinson's disease, amyotrophic lateral sclerosis , Diabetes, Huntington, Prion disease, or a tumor.

### FIGURES

Fig. 1 shows an ATR-IR difference spectrum before (grey curve) and after NHS-activation (black curve) of the azide-functionalized surface with DBCO-NHS-ester as described in the example.
Fig. 2 shows an IR spectrum of the azide-labeled antibody as described in the example.
Fig. 3 shows an IR spectrum and kinetic profile of selected IR bands recorded during casein fragment blocking of NHS-activated surface as described in the example.
Fig. 4 shows the kinetic profile of the amide 2 band in both stability tests in comparison (light grey squares: inventive example; black circles: comparative example).
Fig. 5 shows the relative amount of blocking material on each of the surfaces in both stability tests during washing (light grey squares: inventive example; black circles: comparative example).
Fig. 6 shows IR spectra for both inertness tests (light grey graph: inventive example; black graph: comparative example).
Fig. 7 shows IR spectra which were recorded after the start of the wash step in the activity test D (grey graph: inventive example; black graph: comparative example).

### EXAMPLES

In the following, the invention is further described by specific examples. The examples are not meant to be construed as limiting the invention in any way.

### A. Molecular probe functionalized element according to one embodiment invention and process for preparing the same

- Solid base element:: Silicon ATR Crystal (internal reflection element), trapezoidal, incidence angle 45°, 52mm x 20mm x 2mm (Tol: +/- 0.1 mm), optically polished
- Linker A1:: 1-azido-N-(3-(triethoxysilyl)propyl)-3,6,9,12,15-pentaoxaoctadecan-18-amide
- Matrix precursor compound:: 3-(2-(2-methoxyethoxy)ethoxy)-N-(3-(triethoxysilyl)propyl)propanamide
- Linker A2:: 2,5-dioxopyrrolidin-1-yl 6-{2-azatricyclo[10.4.0.0^{4,9}]hexadeca4,6,8,12,14,16-hexaen-10-yn-2-yl}-6-oxohexanoate
- Peptide compound:: casein fragment blocking solution, made from bovine casein (Sigma Aldrich / Art.-Nr.: C3400) by alkaline hydrolysis
- Linker B1:: 2,5-dioxopyrrolidin-1-yl 1-{2-azatricyclo[10.4.0.0^{4,9}]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate
- Linker B2:: 2,5-dioxopyrrolidin-1-yl 1-azido-3,6,9,12,15,18,21,24-octaoxaheptacosan-27-oate
- Molecular probe:: anti-p-Amyloid (13-28) antibody, mouse monoclonal, clone BAM90.1, purified from hybridoma cell culture (Sigma Aldrich / Art.Nr.: A8978)
- Device:: ATR-IR-biosensor
- Target analyte:: Amyloid-beta(13-28)-BSA conjugate

### Pretreatment of the silicon crystal (process step a))

The surface was activated by an oxidative treatment to produce a stable silicon dioxide layer.

### Silanization of a silicon crystal (process step b))

For silanization of the silicon crystal, a mixture of linker A1 and the matrix precursor compound in 2-propanol was prepared and contacted with a selected surface of the silicon crystal. The crystal was subsequently rinsed with H₂O and dried under nitrogen. After completion of the silanization, the ATR-FTIR difference spectrum (before and after silanization) shows an azide band at 2110 cm⁻¹.

### NHS activation of the azide moieties of the silanized surface (process step b))

Linker A2 was reacted in a SPAAC click reaction with the previously silanized crystal surface. As understood by those skilled in the art, the coupling takes place by reaction of the DBCO group with the azide group on the silanized surface. After SPAAC click reaction, reactive NHS groups are available on the silanized silicon crystal which can be reacted with peptide compounds from the casein fragment blocking solution.

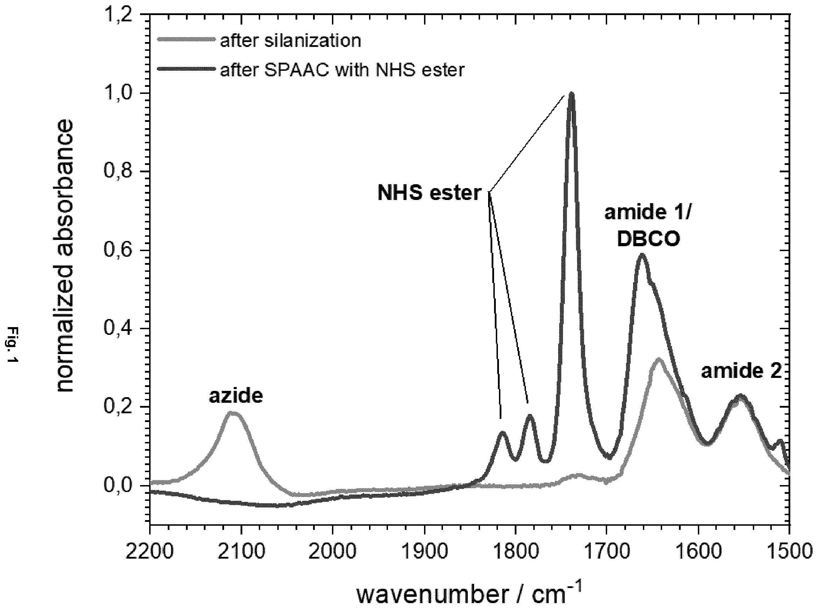

After completion of the SPAAC click reaction, the ATR-IR difference spectrum (before silanization / after click reaction) showed 3 NHS bands at 1738 cm⁻¹, 1782 cm⁻¹, and 1815 cm⁻¹. The azide band at 2110 cm⁻¹ has disappeared in the difference spectrum. The ATR-IR spectrum is shown in Fig. 1.

### Production of azide-labeled antibody (preparation for process step e))

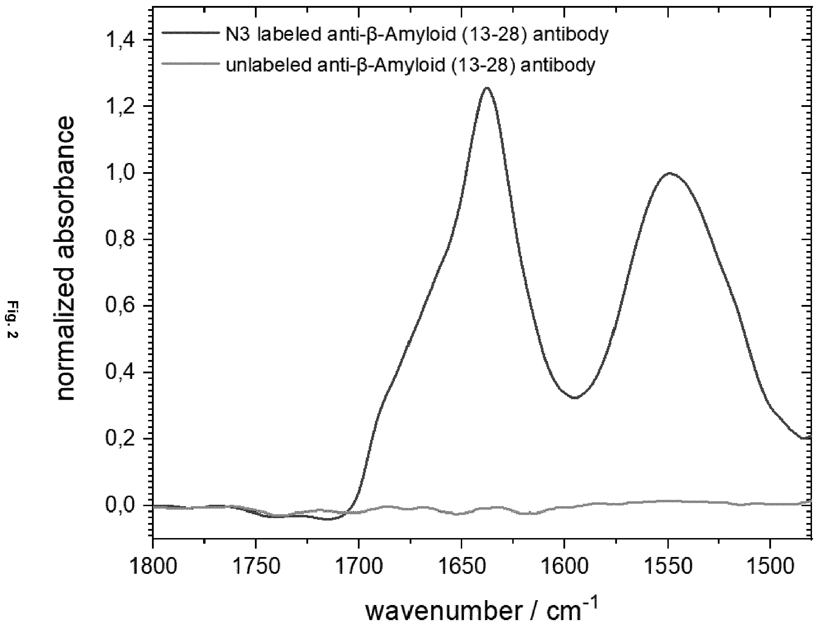

The anti-amyloid-beta antibody was reacted with linker B2 for preparing an azide-labeled anti-amyloid-beta antibody. An IR spectrum of the azide-labeled antibody is shown in Figure 2.

### Production of Aβ-13-28-BSA conjugate

1-Ethyl-3-(3-dimethylaminopropyl)carbodiimide solution and hydroxy-2,5-dioxopyrrolidine-3-sulfonic acid was mixed with 1 ml of BSA solution (10 mg/ml) (Sigma Aldrich, Art. No.:A9413) to achieve a 14-fold molar excess of both substances followed by incubation for 15 min at room temperature. After buffer exchange, 0.5 ml of Aβ13-28 (2 mg/ml in PBS buffer) was added to the activated BSA. This is followed by incubation for 2h at room temperature and a subsequent preparative gel chromatography.

### Blocking of surface and immobilization of antibody on blocking layer (process steps c) to e))

After silanization and NHS-activation by the SPAAC click reaction (i.e. process step b)), a protein-reactive silicon crystal was available. In the subsequent surface functionalization, the NHS-functionalized crystal surface was first blocked with the casein fragment solution. Covalent bonding can be achieved via amide bonding of the primary amine group of the lysines of the casein peptides or the N-termini of the casein peptides with the NHS-functional groups of the silicon crystal.

The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with PBS buffer. Subsequently the casein fragment blocking solution was circulated over the crystal surface, followed by a washing step with PBS in flow-through mode.

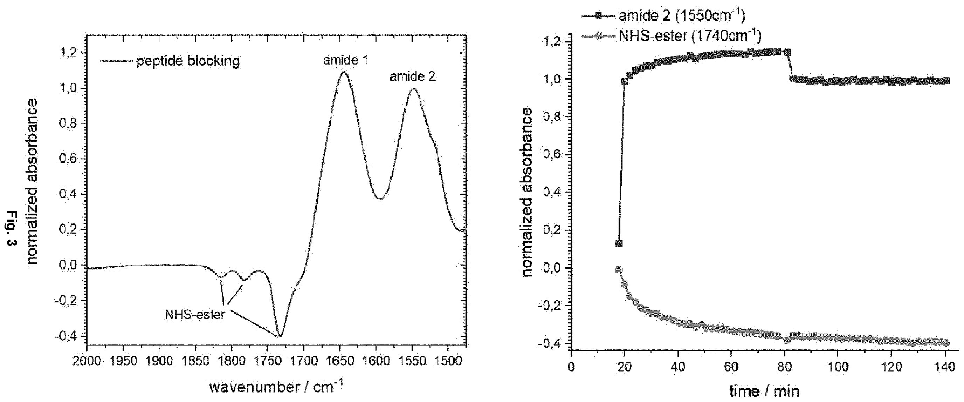

During the blocking process, complete IR spectra were continuously recorded and referenced against the PBS background. The blocking process was monitored by following an increase of the amide 2 absorbance (1550 cm-1) and simultaneously a decrease of NHS absorbance (1740 cm⁻¹) in time. Fig. 3 shows the corresponding IR spectra and kinetics.

Next, excess NHS groups were quenched with a ethanolamine solution followed by another washing step.

Subsequently, the peptide blocking layer was DBCO functionalized by reaction with the NHS-and DBCO-containing linker B1 in flow-through mode.

In a next step, the previously prepared azide-labeled anti-amyloid-beta antibody was immobilized on the DBCO-functionalized peptide blocking layer by a SPAAC click reaction between the DBCO moiety and the azide group in flow-through mode. During antibody attachment, complete IR spectra were continuously recorded and compared with the background before antibody attachment. An indication for successful immobilization of the antibody on the surface is the amide 2 signal (1550 cm1).

The functionalized surface prepared in this process corresponds to a molecular probe functionalized element according to one embodiment of the invention.

### B. Stability tests

Stability tests are exemplary shown for two different peptide-blocked ATR crystal surfaces.

The first stability test was performed with a peptide-blocked ATR silicon crystal surface which was prepared by: (1) providing a NHS-activated surface prepared by the process according to the invention (steps a) and b) of the inventive example as described above); (2) blocking the NHS-activated surface with the casein fragment blocking solution (step c) of the inventive example as described above). For the stability tests, the peptide-blocked ATR crystal surface was not linked to a molecular probe.

The second stability test was performed with a peptide-blocked ATR crystal surface which was prepared using the NHS-silane-functionalization method of a germanium crystal as described in the prior art (US patent application US 20200240908 A1), followed by blocking with the same casein fragment solution.

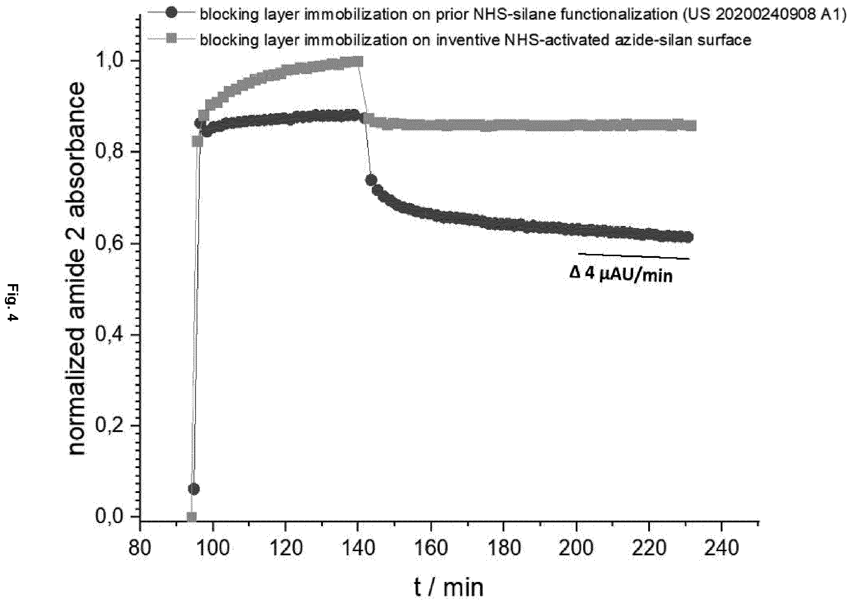

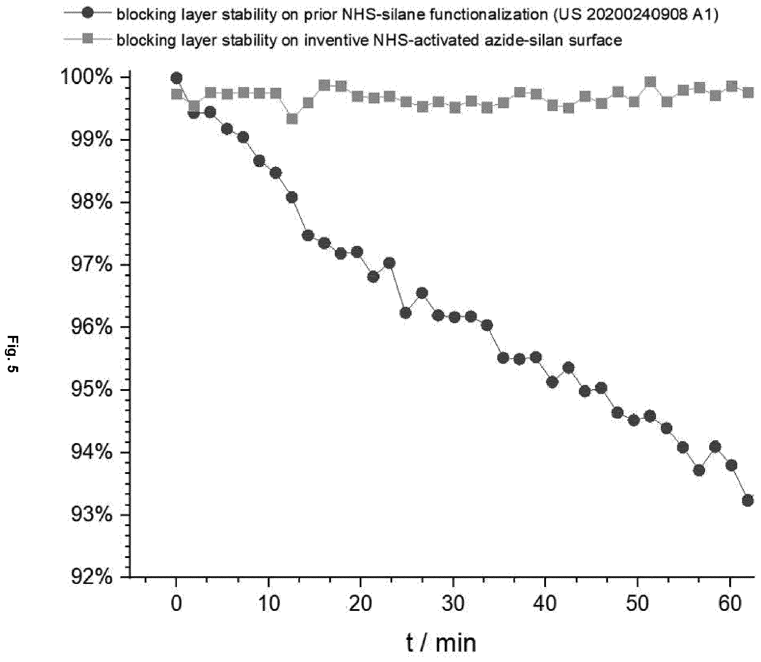

Subsequently, both surfaces were washed with PBS for 2 h in flow-through mode (wash). Continuous IR spectra were recorded during both steps. Results are shown in Figures 4 and 5, wherein the graph "inventive NHS-activated azide-silane surface" corresponds to the surface prepared according to the invention and the graph "prior NHS-silane functionalization" corresponds to the comparative surface prepared by a method of the prior art (US patent application US 20200240908 A1).

Figure 4 shows the kinetic profile of the amide 2 band in both stability tests in comparison. Looking at the kinetic profile of the amide 2 band in the wash step, a clear difference can be observed between the example which has been prepared by steps a to c) of the invention and the comparison. The blocking layer on the NHS-activated surface according to steps a) to c) of the invention remains absolutely stable during the entire 2 hours of the wash step. For the comparative NHS-functionalized surface, an initially very strong loss of blocking material can be observed during the wash step which is followed by constant loss of blocking material of about 4 µAU/min.

Figure 5 further illustrates this fact. It shows the relative amount of blocking material on the surface over the course of one hour of PBS wash. Within one hour, about 7% of the blocking layer is lost on the surface prepared with the prior art method. In comparison, on the surface prepared according to the present invention, nothing or almost nothing of the blocking layer is lost during the entire hour. Due to the fact that an experiment (e.g. further functionalization or eventually detection of an analyte by the molecular probe) performed on the blocked surface takes at least 2 h, a loss of 7% blocking material per hour has a very negative impact on the quality of the experiment.

The stability tests show that the inventive molecular probe functionalized element provides a much more stable functionalized surface which leads to an improved quality for e.g. the detection of a target analyte.

### C. Inertness test

Such in a further exemplary example the peptide-blocked ATR crystal surfaces as described above for the stability test were subjected to an inertness test.

This inertness test was performed on surfaces which were made exactly as described above for the stability test. Subsequently, 300 µl of human CSF (cerebrospinal fluid) was added to the blocked surfaces to observe possible non-specific protein adsorption (NSP adsorption) on the surface. The CSF was circulated over the surfaces for 1 h for this purpose (circ). Subsequently, the surfaces were washed with PBS for 2 h in flow-through mode (wash). During both steps IR spectra were continuously recorded.

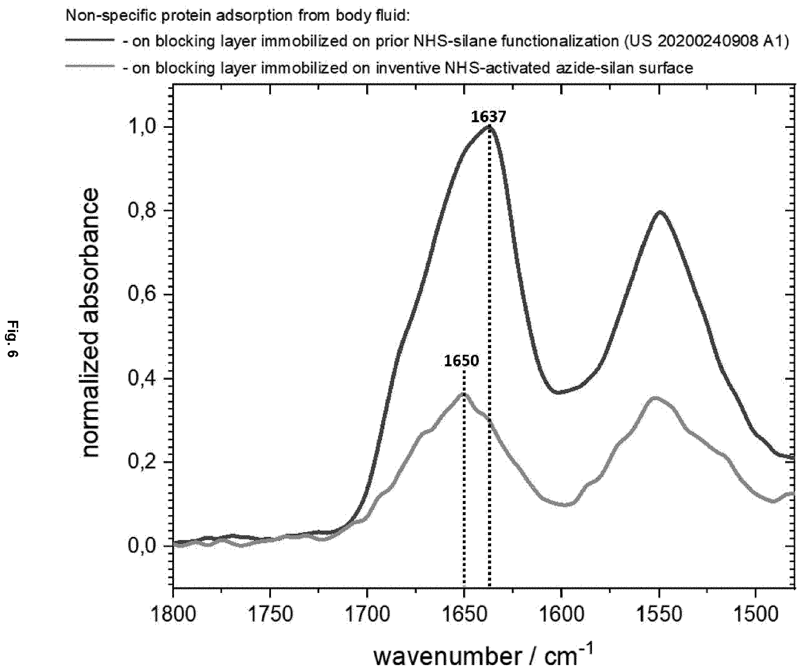

The IR spectra are shown in figure 6, wherein the spectrum "blocking layer immobilized on inventive NHS-activated azide-silane" corresponds to the surface prepared according to steps a) to c) of the invention and the spectrum "blocking layer immobilized on prior NHS-silane functionalization" corresponds to the comparative surface prepared by a method of the prior art (US patent application US 20200240908 A1).

Figure 6 shows that the surface prepared according to steps a) to c) of the invention has significantly improved properties with respect to non-specific protein adsorption from complex body fluids such as CSF. The extent of non-specific protein adsorption is reduced by half. Furthermore, the band shape and the position of the maximum of the amide 1 band indicate that the comparative surface binds significantly more beta-sheet structured proteins in a non-specific manner. In contrast, the surface prepared in accordance with the invention shows only a slight non-specific binding of non-beta sheet structured proteins.

The inertness test shows that the inventive molecular probe functionalized element is much more inert towards non-specific binding of proteins, e.g. from a body fluid, than a comparable functionalized surface. This in turn improves a detection of a target analyte from a complex sample when using the inventive molecular probe functionalized element.

### D. Molecular probe activity - Test of antibody activity

Two antibody activity tests were exemplary performed using (i) the molecular probe functionalized element according to one embodiment of the invention prepared as described in section A above (inventive example), and (ii) using a comparative molecular probe functionalized element (comparative example).

The comparative molecular probe functionalized element was prepared by using a conventional blocking technique, wherein the antibody is first attached to the NHS-activated surface followed by blocking of the surface. The comparative probe element was prepared as follows:
(1) The same process steps a) and b) were carried out to obtain an NHS-activated surface by SPAAC click reaction; (2) covalently binding the anti-amyloid-beta antibody on the surface by NHS-coupling of the antibody with the NHS-activated surface; (3) reacting the remaining NHS groups of the NHS-activated surface with the casein fragment blocking solution.

The steps of antibody binding and surface blocking were analyzed for the inventive example and the comparative example by IR spectroscopy. In the comparative example, the anti-p-Amyloid (13-28) antibody (A8978) was covalently bound at the beginning. In the following blocking step, the blocking peptide was bound to saturate the free surface patches.

In the inventive example, the surface was first inertized by stable blocking with the peptide compound. This was followed by azide-linker immobilization. Subsequently, the linker-modified anti -β-Amyloid (13-28) antibody was immobilized on the surface in two steps.

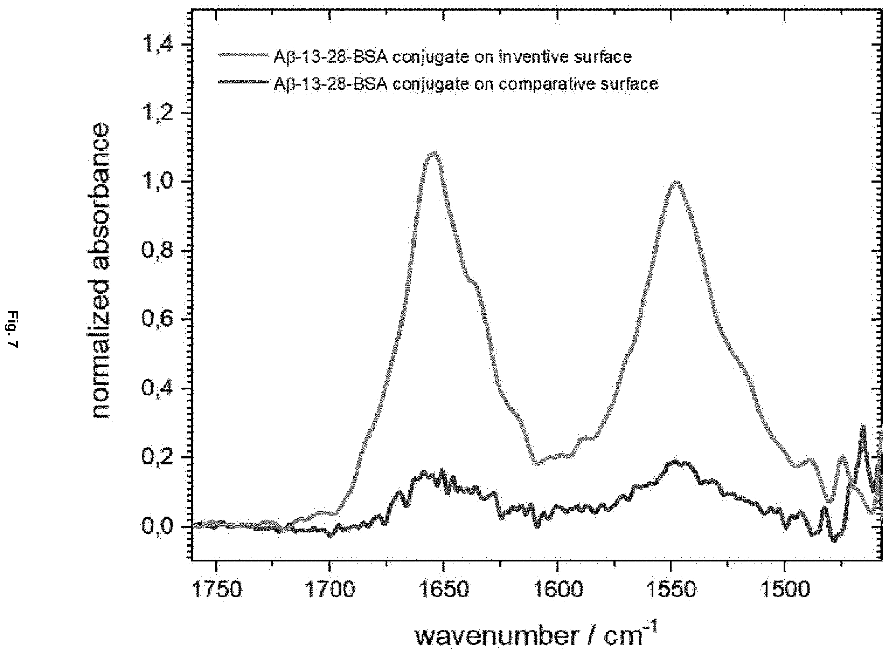

Aβ-13-28-BSA conjugate was circulated over the test surfaces for binding to the immobilized antibody, followed by a washing step. The activity tests show that the molecular probe functionalized element according to one embodiment of the invention (inventive surface) is significantly more active than the comparative molecular probe functionalized element (comparative surface). Figure 7 shows IR spectra which were recorded about 5 minutes after the start of the wash step. The increase in activity for the inventive example can be clearly seen in the spectra. In addition, it can be seen that with the gain in signal intensity with no change in noise, a significantly improved signal-to-noise ratio is achieved in the inventive example. The spectra show an amide 1 band maximum of 1655 wavenumbers as expected for the antigen in use.

## Claims

1. A molecular probe functionalized element comprising
a solid base element,
a first layer comprising a linker A,
a second layer comprising a peptide compound,
a third layer comprising a linker B, and
a fourth layer comprising a molecular probe,
wherein the peptide compound is covalently bound to the solid base element via linker A and the molecular probe is covalently bound to the peptide compound via linker B.

2. The molecular probe functionalized element according to claim 1, wherein the solid base element is an IR-transparent optical element, and preferably an attenuated total reflection infrared (ATR-IR) waveguide.

3. The molecular probe functionalized element according to claim 1 or 2, wherein at least a part of the solid base element has an oxide surface layer, and the peptide is covalently bound to the oxide surface layer via linker A.

4. The molecular probe functionalized element according to any one of the preceding claims, wherein linker A comprises a surface attachment group which is covalently bound to the solid base element, and wherein the surface attachment group is a silicon-containing group or a sulfurcontaining group.

5. The molecular probe functionalized element according to any one of the preceding claims, wherein linker A comprises
a surface attachment group which is covalently bound to the solid base element,
a peptide attachment group which is covalently bound to the peptide compound,
wherein the surface attachment group is covalently bound to the peptide attachment group via a linking moiety, and wherein the linking moiety comprises a -(OCH₂CH₂)ₖ- group, wherein k is an integer from 1 to 100, preferably from 1 to 50, and more preferably from 1 to 16.

6. The molecular probe functionalized element according to any one of the preceding claims, wherein linker A comprises
a surface attachment group which is covalently bound to the solid base element, and
a peptide attachment group which is covalently bound to the peptide compound,
wherein the surface attachment group is covalently bound to the peptide attachment group via a linking moiety,
wherein the linking moiety comprises a group which is obtainable by a bioorthogonal ligation reaction, and preferably an optionally substituted N-heterocyclic group selected from triazoles, dihydropyridazines and pyridazines.

7. The molecular probe functionalized element according to any one of the preceding claims, wherein the first layer further comprises a matrix compound, which is covalently bound to the solid base element, wherein the matrix compound is:
(i) a PEG-containing or mPEG-containing silane, and/or
(ii) an optionally substituted alkyl sulfide.

8. The molecular probe functionalized element according to any one of the preceding claims, wherein the peptide compound of the second layer is selected from the group consisting of peptides, substituted peptides, proteins, protein fragments, and mixtures thereof, or
wherein the second layer comprises a mixture of peptide compounds, wherein the mixture of peptide compounds is a mixture of peptides obtainable or obtained from a protein hydrolysate, and wherein the peptides are optionally substituted.

9. The molecular probe functionalized element according to any one of the preceding claims, wherein linker B comprises
a peptide attachment group which is covalently bound to the peptide compound,
a molecular probe attachment group which is covalently bound to the molecular probe, and
wherein the peptide attachment group is covalently bound to the molecular probe attachment group by a linking moiety, and
wherein the linking moiety comprises a group which is obtainable by a bioorthogonal ligation reaction, and preferably an optionally substituted N-heterocyclic group, wherein the N-heterocyclic group is selected from triazoles (e.g. 1,2,3-triazoles), dihydropyridazines, and pyridazines.

10. The molecular probe functionalized element according to any one of the preceding claims, wherein the molecular probe is an antigen, an antibody, a fragment of an antibody, an antibody fusion protein or fusion proteins with antibody fragments, an antibody conjugate or a conjugate with an antibody fragment, a protein complex which optionally contains an antibody fragment or a fusion protein thereof, an anticalin, a nanobody, a small organic molecule, a drug, a nucleic acid, an aptamer, a lipid, a carbohydrate, a peptide, or a mixture thereof.

11. The molecular probe functionalized element according to any one of the preceding claims, wherein the molecular probe is capable of forming a complex with a target analyte selected from the group consisting of amyloid-beta (Aβ) peptides and isoforms thereof, alpha synuclein, tau protein, TDP-43, human islet amyloid polypeptide (hIAPP), Prion protein, and p53.

12. A device, preferably a biosensor, wherein the device comprises a molecular probe functionalized element according to any one of claims 1 to 11.

13. A process for preparing a molecular probe functionalized element according to any one of claims 1 to 11,
wherein the process comprises the steps of:
a) providing a solid base element;
b) preparing a linker A precursor which is covalently bound to the surface of the solid base element;
c) reacting the linker A precursor with a peptide compound to covalently bind the peptide compound to the solid base element via linker A;
d) preparing a linker B precursor which is covalently bound to the peptide compound; and
e) reacting the linker B precursor with a molecular probe to covalently bind the molecular probe to the peptide compound via linker B.

14. A method for detecting a biomarker; comprising
a) bringing into contact the molecular probe functionalized element according to any one of claims 1 to 11 with a sample suspected to comprise a target analyte, and optionally wherein the sample is a sample of intestinal fluid, ocular fluid, pulmonary fluid, blood or CSF;
b) detecting the biomarker based on an interaction between the molecular probe and the target analyte, and optionally wherein the interaction between the molecular probe and the target analyte is detected by infrared spectroscopy, and optionally wherein the biomarker is an amide band maximum of the target analyte.

15. Use of a molecular probe functionalized element according to any one of claims 1 to 11 or of a device according to claim 12 for one or more of:
a companion diagnostic test;
diagnosing Alzheimer's disease, Parkinson's disease, amyotrophic lateral sclerosis, Diabetes, Huntington, Prion disease, or a tumor in a patient;
monitoring of therapy of patients with Alzheimer's disease, Parkinson's disease, amyotrophic lateral sclerosis , Diabetes, Huntington, Prion disease, or a tumor; and
screening of drugs for the treatment of Alzheimer's disease, Parkinson's disease, amyotrophic lateral sclerosis , Diabetes, Huntington, Prion disease, or a tumor.
